# EUROPEAN PATENT SPECIFICATION

(11) **EP 3 060 676 B1**
(45) Date of publication and mention of the grant of the patent: **20.11.2019**
(21) Application number: 14790471.8
(22) Date of filing: 20.10.2014
(51) Int. Cl.: C12Q 1/02, C12Q 1/04, C12Q 1/26, C12M 1/34, C12M 1/00

(54) **SELF-CONTAINED ANAEROBIC ENVIRONMENT-GENERATING CULTURE DEVICES AND METHODS OF USE**
KULTURVORRICHTUNGEN ZUR ERZEUGUNG AUTONOMER ANAEROBER UMGEBUNGEN UND VERFAHREN ZUR VERWENDUNG
DISPOSITIFS AUTONOMES DE CULTURE GÉNÉRATEURS D'UN ENVIRONNEMENT ANAÉROBIE, ET PROCÉDÉS D'UTILISATION

(30) Priority: 24.10.2013 US 201361895170 P
(43) Date of publication of application: 31.08.2016
(73) Proprietor: 3M Innovative Properties Company, St. Paul, MN 55133-3427 (US)
(72) Inventor: BJORK, Jason, W., Saint Paul, MN 55133-3427 (US); CELT, Mara, S., Saint Paul, MN 55133-3427 (US); STANENAS, Adam, J., Saint Paul, MN 55133-3427 (US)
(74) Representative: Mathys & Squire
(86) International application number: PCT/US2014/061351
(87) International publication number: WO 2015/061213

(56) References cited:
- EP-A2- 0 246 912
- JP-A- 2006 061 067
- US-A- 5 955 344
- US-A1- 2009 280 522

## Description

### CROSS REFERENCE TO RELATED APPLICATIONS

This application claims priority to U.S. Provisional Patent Application No. 61/895,170, filed October 24, 2013.

### BACKGROUND

Many bacteria are sensitive to oxygen and will not grow in its presence. It can be useful in various environments to determine the viability of such anaerobic microorganisms. For example, it can be important to determine if anaerobic microorganisms are present in food processing and/or packaging facilities. It can also be important to determine the presence of anaerobic microorganisms in medical environments, for example, to determine the presence of pathogens in diagnostic assays. As another example, water treatment facilities test water samples to determine the presence or absence of such microbes.

A variety of devices are available for culturing microorganisms. For example, microorganisms have long been cultured using Petri dishes. As known in the art, Petri dishes are round, shallow, flat bottomed dishes with a suitable medium for growth of the microorganism, such as agar and nutrients. The use of agar medium, however, can be inconvenient and time consuming. For example, agar medium must be sterilized, melted and cooled prior to addition of the sample.

In addition, it can be difficult to provide an environment suitable for culturing anaerobic microorganisms using Petri dishes. Because anaerobic microorganisms do not thrive in the presence of oxygen, cumbersome physical and chemical techniques can be required to grow such organisms. Typically, such devices must be modified, i.e., shaped or configured, to provide a physical barrier to the transmission of oxygen.

Other techniques have been developed that use chemical agents incorporated into an anaerobic culturing device to remove oxygen. Generally, such devices include a reducing agent or sterile membrane fragments of bacteria incorporated into a gel or nutrient media. In addition, U.S. Patent No. 3,338,794 describes an anaerobic bacteria culturing device formed of oxygen impermeable film layers and a nutrient media between the films, which includes a reducing compound.

These and other devices, however, can also be cost prohibitive and may not be readily disposable. These devices can also be cumbersome to assemble and/or use. Although attempts have been made to produce a simple device for culturing anaerobic microorganisms in an aerobic environment, there remains a need for improved anaerobic culture devices.

US 5,955,344 discloses an apparatus for growing anaerobic microorganisms including a liquid medium.

US 2009/0280522 A1 discloses a method and system for the detection of growth and metabolism of a cellular microorganism in a population of microorganisms in a non-liquid, culture medium.

EP 0 246 912 A2 discloses a method and compositions for protecting and transporting anaerobic microorganisms, but not for culturing.

### SUMMARY

In general, the present disclosure relates to detection and, optionally, enumeration of microorganisms in a sample. In particular, the present disclosure relates to growth and detection of microaerotolerant, microaerophilic, or obligately-anaerobic microorganisms. It is now known this growth and detection can be conducted using a self-contained anaerobic environment-generating culture device.

The inventive culture device and methods disclosed herein provide for growth, detection, and differentiation of microaerotolerant, microaerophilic, or obligately-anaerobic microorganisms (e.g., bacteria, yeast) even while incubating the microorganisms in oxygen-containing (e.g., normal atmospheric oxygen-containing) environments. Advantageously, this eliminates the need for specialized incubation equipment and reagents (e.g., anaerobe jars, single-use anaerobe sachets, palladium catalysts, anaerobic glove boxes) that are typically required to culture anaerobic microorganisms. Additionally, the inventive methods provide for differentiation of bacteria by permitting detecting the production of carbon dioxide gas from individual colonies, thus eliminating the additional incubation time needed for isolation of pure cultures and the use of fermentation tubes to detect gas production. Furthermore, the disclosure relates to the enumeration of microaerotolerant, microaerophilic, or obligately-anaerobic bacteria in a sample. Microaerotolerant, microaerophilic and obligately-anaerobic microorganisms share the common feature that they require reduced-oxygen environments in which to grow and reproduce.

Disclosed is a method of detecting a microorganism in a sample. The method can comprise contacting a growth region of a self-contained anaerobic environment-generating culture device with a predefined volume of aqueous liquid wherein, prior the step of contacting the growth region with the predefined volume, the growth region comprises a dry, cold-water-soluble gelling agent and an effective amount of a substantially dry enzyme component of an enzyme-mediated oxygen depletion system. The method further can comprise contacting the growth region with a sample, incubating the culture device for a period of time sufficient to permit formation of a microbial colony, and detecting the microbial colony.

The present invention provides a method of detecting a microorganism in a sample. The method comprises contacting a growth region of a self-contained anaerobic environment-generating culture device with a predefined volume of aqueous liquid wherein, prior the step of contacting the growth region with the predefined volume, the growth region comprises a dry, cold-water-soluble gelling agent and a fermentable carbohydrate, wherein the growth region is defined as a volume disposed between the inner surfaces of a first substrate and a second substrate, and wherein the dry, cold water-soluble gelling agent is disposed as a first coating in the growth region. The method further comprises depositing an effective amount of an enzyme component of an enzyme-mediated oxygen depletion system into the growth region, contacting the growth region with a sample, incubating the culture device for a period of time sufficient to permit formation of a microbial colony, and detecting the presence or absence of a microbial colony.

Also disclosed is a method of detecting a microorganism in a sample. The method can comprise contacting a growth region of a self-contained anaerobic environment-generating culture device with a predefined volume of aqueous liquid wherein, prior the step of contacting the growth region with the predefined volume, the growth region comprises a dry, cold-water-soluble gelling agent. The method further can comprise depositing an effective amount of an enzyme substrate component of an enzyme-mediated oxygen depletion system into the growth region, contacting the growth region with a sample, incubating the culture device for a period of time sufficient to permit formation of a microbial colony, and detecting the microbial colony. In any embodiment, the method further can comprise depositing an effective amount of an enzyme component of the enzyme-mediated oxygen depletion system into the growth region.

In another aspect, the present invention provides a culture device for enumerating colonies of microorganisms. The culture device comprises a first substrate having opposing inner and outer surfaces, a second substrate having opposing inner and outer surfaces, a growth region disposed between the inner surfaces of the first and second substrates, a first effective amount of a substantially dry enzyme component of an enzyme-mediated oxygen depletion system, a second effective amount of a substantially dry enzyme substrate component of the enzyme-mediated oxygen depletion system, and a dry, cold-water-soluble gelling agent disposed in the growth region. The first effective amount is disposed in a first coating in the growth region. The second effective amount is disposed in a second coating in the growth region. The first substrate and second substrate care substantially non-transmissible to gaseous oxygen. Components that are substantially dry have a water content no greater than about the water content of the dehydrated coating once it has been permitted to equilibrate with the ambient environment. The first and second coatings are placed in fluid communication when an aqueous liquid is deposited between the first and second substrates.

Further disclosed is a method of making a culture device for culturing anaerobic microorganisms. The method can comprise depositing a first coating onto a portion of a first substrate, the first coating being formed using a liquid mixture comprising a liquid and an effective amount of an enzyme component of an enzyme-mediated oxygen depletion system; drying the first coating; depositing a second coating onto a second substrate, the second coating comprising an enzyme substrate component of the enzyme-mediated oxygen depletion system; and positioning the first substrate adjacent the second substrate such that the first coating faces the second coating and such that a growth region disposed between the first substrate and the second substrate overlaps a portion of the first coating and a portion of the second coating.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

The terms "comprises" and variations thereof do not have a limiting meaning where these terms appear in the description and claims.

As used herein, "a," "an," "the," "at least one," and "one or more" are used interchangeably. Thus, for example, a nutrient can be interpreted to mean "one or more" nutrients.

The term "and/or" means one or all of the listed elements or a combination of any two or more of the listed elements.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

Additional details of these and other embodiments are set forth in the accompanying drawings and the description below. Other features, objects and advantages will become apparent from the description and drawings, and from the claims.

### BRIEF DESCRIPTION OF DRAWINGS

FIG. 1 is a top perspective view, partially in section, of one embodiment of a self-contained anaerobic environment-generating culture device according to the present disclosure.
FIG. 2 is a top perspective view of an alternative embodiment of a self-contained anaerobic environment-generating culture device according to the present disclosure.
FIG. 3 is a cross-sectional view of the culture device of FIG. 2, the culture device shown with the first substrate 12 and second substrate 18 separated in order to deposit a liquid and/or a sample into the growth region of the culture device.
FIG. 4 is a graph of the oxygen concentration versus time in culture devices, some of which received an enzyme-mediated oxygen depletion system therein.
FIG. 5 is a graph of the oxygen concentration versus time in several self-contained anaerobic environment-generating culture device into which various effective amounts of enzyme substrate were received.

### DETAILED DESCRIPTION

Before any embodiments of the present disclosure are explained in detail, it is to be understood that the invention is not limited in its application to the details of construction and the arrangement of components set forth in the following description or illustrated in the following drawings. The invention is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "connected" and "coupled" and variations thereof are used broadly and encompass both direct and indirect connections and couplings. Further, "connected" and "coupled" are not restricted to physical or mechanical connections or couplings. It is to be understood that other embodiments may be utilized and structural or logical changes may be made without departing from the scope of the present disclosure. Furthermore, terms such as "front," "rear," "top," "bottom," and the like are only used to describe elements as they relate to one another, but are in no way meant to recite specific orientations of the apparatus, to indicate or imply necessary or required orientations of the apparatus, or to specify how the invention described herein will be used, mounted, displayed, or positioned in use.

The present disclosure generally relates to detection and, optionally, enumeration of microorganisms in a sample. In particular, the present disclosure relates to growth and detection of microaerotolerant, microaerophilic, or obligately-anaerobic microorganisms. It is now known this growth and detection can be conducted using a self-contained anaerobic environment-generating culture device.

It is now known that a dry, rehydratable self-contained anaerobic environment-generating culture device can be made. The culture device comprises effective amounts of a substantially-dry enzyme component and a substantially-dry enzyme substrate component, the components being disposed in a growth zone of the culture device and being capable of rehydration in a predetermined volume of aqueous solution wherein, upon rehydration, the components are capable of participating in an oxygen-consuming reaction. Further, it is now known the oxygen-consuming reaction can consume enough oxygen to facilitate growth of a microaerotolerant microorganism, a microaerophilic microorganism, or an obligately-anaerobic microorganism. Furthermore, the culture device can be held in an aerobic environment wherein the culture device can maintain a reduced oxygen environment for at least about eight days in order to facilitate growth of the aforementioned microorganisms.

Bacterial species of interest can be analyzed in a test sample that may be derived from any source, such as a physiological fluid, e.g., blood, saliva, ocular lens fluid, synovial fluid, cerebral spinal fluid, pus, sweat, exudate, urine, mucus, mucosal tissue (e.g., buccal, gingival, nasal, ocular, tracheal, bronchial, gastrointestinal, rectal, urethral, ureteral, vaginal, cervical, and uterine mucosal membranes), lactation milk, feces or the like. Further, the test sample may be derived from a body site, e.g., wound, skin, anterior nares, nasopharyngeal cavity, nasal cavities, anterior nasal vestibule, scalp, nails, outer ear, middle ear, mouth, rectum, vagina, axilla, perineum, anus, or other similar site.

Besides physiological fluids, other test samples may include other liquids as well as solid(s) dissolved or suspended in a liquid medium. Samples of interest may include process streams, water, food, food ingredients, beverages, soil, plants or other vegetation, air, surfaces (e.g., walls, floors, equipment, utensils in a manufacturing plant, hospital, clinic, or home, for example), and the like.

Non-limiting examples of bacteria that require (i.e., microaerophilic bacteria) and/or tolerate (i.e., aerotolerant bacteria) reduced-oxygen tension environments in which to grow include *Helicobacter pylori, Campylobacter* species (e.g., *C. jejuni, C. coli, C. fetus*), *Streptococcus intermedius, Streptococcus sanguis, Streptococcus constellatus, Gemella morbillorum, Lactobacillus* species, and *Streptococcus pyogenes.*

Anaerobic bacteria are ubiquitous in nature. The anaerobic bacteria can be obligately-anaerobic or, alternatively can be facultatively-anaerobic. Nonlimiting examples of obligately-anaerobic bacteria include *Actinomyces* species, *Clostridium* species (e.g., C. *perfringens, C. tetani, C sporogenes, C. botulinum, C. difficile, C. butyricum, C. acetobutylicum*)*,* lactic-acid bacteria (e.g., *Lactobacillus* species, *Leuconostoc* species, *Pediococcus* species, *Lactococcus* species), *Bacteroides* species (e.g., *B fragilis*)*,* and *Peptostreptococcus* species (e.g., *P micros, P. magnus, P. asaccharolyticus, P. anaerobius,* and *P. tetradius).* Nonlimiting examples of facultatively-anaerobic bacteria include *Enterobacteria* (e.g., *E. coli, Salmonella* species, *Citrobacter freundii, Staphylococcus aureus,* and *Listeria* species (e.g., *L. monocytogenes*).

Because many yeast species are facultatively-anaerobic and some yeast species are obligately anaerobic, it is also contemplated that the self-contained anaerobic environment-generating culture device of the present disclosure is useful for growth and detection of yeast microorganisms.

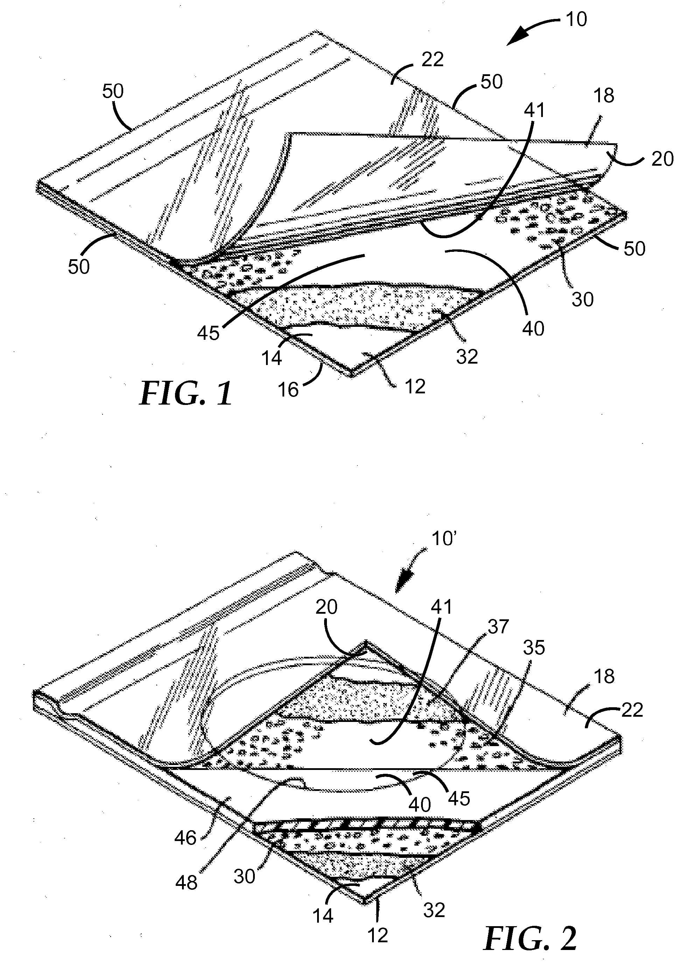

In one aspect, the present disclosure provides a culture device for culturing and detecting a microorganism that grows in reduced-oxygen environments. With reference to FIG. 1, a self-contained anaerobic environment-generating culture device 10 of the present disclosure comprises a waterproof first substrate 12, a waterproof second substrate 18, and a growth region 45 disposed between the first substrate 12 and second substrate 18. The first substrate 12 has an inner surface 14 and an outer surface 16 opposite the inner surface. The second substrate 18 has an inner surface 20 and an outer surface 22 opposite the inner surface. In any embodiment, the inner surface 14 of the first substrate 12 is disposed in facing relationship with the inner surface 20 of the second substrate 18.

Disposed on the inner surface 14 of the first substrate 12 is a first dry coating 30. The first dry coating 30 is fixed to and covers at least the growth region 45 of the inner surface 14 of the first substrate 12. The first dry coating 30 comprises a dry, cold-water-soluble gelling agent and a component of an enzyme-mediated oxygen depletion system. In any embodiment, the component in the first coating comprises a first effective amount of a substantially dry enzyme component of the enzyme-mediated oxygen depletion system. Alternatively, in any embodiment, the component in the first coating comprises a second effective amount of a substantially dry enzyme substrate capable of reacting with the enzyme component in a reaction that utilizes oxygen. The first dry coating 30 can cover the entire inner surface 14 of the first substrate 12, but preferably covers at least the part of the inner surface 14 that defines at least a first portion 40 of the growth region 45 of the culture device 10. The growth region 45 is the interior part of the culture device 10 that holds the sample during inoculation of the device with a sample and during growth and detection of microorganisms, if present in the sample.

In the illustrated embodiment of FIG. 1, the first dry coating 30 covers substantially all of the inner surface 14 of the first substrate 12. Thus, the growth region can be at any accessible location in the culture device 10 between the first substrate 12 and the second substrate 18. Preferably, the growth region is located away from the peripheral edges 50.

The growth region 45 is defined as a volume disposed between the inner surfaces (inner surfaces 14 and 20, respectively) of the first substrate 12 and second substrate 18, the volume encompassing at least a portion of the first dry coating 30 and/or second dry coating (discussed below). Thus, when an aqueous liquid is distributed into the growth region, the aqueous liquid is in fluidic contact with at least a portion of the first dry coating 30 and/or second dry coating. The thickness of the growth region 45 may vary depending upon, for example, the volume of aqueous liquid (not shown) deposited in the culture device, the presence of solids (e.g., suspended particulates and/or a membrane filter) associated with the sample (not shown), and/or a spacer means (discussed below), if present, in the culture device 10.

First substrate 12 is preferably a relatively stiff waterproof film made of a material (e.g., polyester, polypropylene, or polystyrene) that will not absorb or otherwise be adversely affected by water. First substrate 12 preferably is made using a material that is substantially nontransmissible to gaseous oxygen. Nonlimiting examples of suitable materials for first substrate 12 include polyester films at least about 15 µm to at least about 180 µm thick, polypropylene films at least about 100 µm to at least about 200 µm thick and polystyrene films at least about 300 µm to about 380 µm thick. Other suitable first substrates include ethylene vinyl alcohol copolymer films, polyvinyl alcohol films, and polyvinylidene chloride films. First substrate 12 can be transparent if one wishes to view colonies through the first substrate.

The second substrate 18 is used to cover the inner surface 14 of the first substrate 12, to define the growth region 45 and, optionally, to view the growth region during shipping, storage, incubation, and/or colony counting. Second substrate 18 is preferably a relatively stiff waterproof film made of a material (e.g., polyester, polypropylene, or polystyrene) that will not absorb or otherwise be adversely affected by water. Second substrates 18 are preferably transparent in order to facilitate the counting of colonies without opening the culture device 10, and are substantially impermeable to microorganisms and water vapor.

Generally, second substrates can be made of materials such as those used to make first substrate 12. Second substrate 18 preferably is made using a material that is substantially nontransmissible to gaseous oxygen. Nonlimiting examples of suitable materials for first substrate 12 include polyester films at least about 15 µm to at least about 180 µm thick, polypropylene films at least about 100 µm to at least about 200 µm thick and polystyrene films at least about 300 µm to about 380 µm thick. Other suitable first substrates include ethylene vinyl alcohol copolymer films, polyvinyl alcohol films, and polyvinylidene chloride films. As shown in FIG. 1, the second substrate 18 can be attached in a hinge-like fashion (e.g., using double-sided adhesive tape) along one edge of the inner surfaces of each of the first substrate 12 and second substrate 18.

A person having ordinary skill in the art will recognize the transmissibility of oxygen gas through a given type of polymer film can be reduced by increasing the thickness of the polymer film. In any embodiment, the first substrate and second substrate of the present disclosure are polymeric films having a suitable thickness to be substantially nontransmissible to gaseous oxygen.

Preferably, when the first coating 30 consists primarily of dry powder or dry powder agglomerate, the first coating is disposed on an adhesive layer 32 that is disposed on at least a portion of the inner surface 14 of the first substrate 12. The first dry coating 30 can be deposited onto the first substrate 12 or onto the optional adhesive layer 32 using compounding processes, adhesive coating processes, and liquid-coating processes and/or dry-coating processes described, for example, in U.S. Patent Nos. 4,565,783; 5,089,413; and 5,232,838.

As regards the first dry coating 30, the coating optionally can comprise any nutrient or nutrient medium that is cold-water-reconstitutable, that does not substantially interfere with the cold-water gelling properties of the gelling agent, and that supports growth of an anaerobic microorganism. The particular nutrient or nutrients suitable for use in the culture device will depend on the microorganism to be grown in the device, and will be easily selected by those skilled in the art. Generally, such nutrients are cold-water soluble. Suitable nutrients for supporting bacterial growth are known in the art and include without limitation yeast extract, peptone, sugars, suitable salts, and the like. In any embodiment, the first dry coating further can comprise a selective agent (e.g., a nutrient, an antibiotic, and combinations thereof) that facilitates the growth of a particular anaerobic microorganism or group of microorganisms over another microorganism or group of microorganisms. Those skilled in the art will recognize that a variety of other formulations could be used and that these do not detract from the scope of this invention.

Suitable gelling agents for use in the first dry coating 30 include cold-water-soluble natural and synthetic gelling agents. Natural gelling agents such as algin, carboxymethyl cellulose, tara gum, hydroxyethyl cellulose, guar gum, locust bean gum, xanthan gum, and synthetic gelling agents such as polyacrylamide, polyurethane, polyethylene oxides, polyvinyl alcohol, and mixtures thereof are generally suitable. Appropriate gelling agents can be selected according to the teaching of this disclosure and the disclosures of U.S. Patent Nos. 4,565,783; 5,089,413; and 5,232,838. Preferred gelling agents include guar gum, locust bean gum, and xanthan gum; these gelling agents being useful individually, or preferably, in combination with one another.

Culture devices of the present disclosure comprise at least one substantially dry component of an enzyme-mediated oxygen depletion system disposed therein. The at least one dry component is hydrated with an aqueous liquid before, during, or after the introduction (e.g., inoculation) of sample material into the growth region of the culture device, as described herein. Typically, the sample material and/or aqueous liquid is introduced into the growth region of the culture device in ambient conditions (i.e., in an aerobic gaseous environment). Thus, after inoculation of the growth region with a sample under aerobic conditions, the aqueous liquid in the growth region of the culture device comprises a first dissolved-oxygen concentration. The enzyme-mediated oxygen depletion system in the culture device functions to reduce the first dissolved-oxygen concentration in the aqueous liquid in the growth region to a second dissolved-oxygen concentration that is substantially lower than the first dissolved-oxygen concentration. This reduction of the dissolved oxygen concentration in the growth region of the inoculated culture device facilitates the growth of an anaerobic or microaerophilic microorganism in the culture device.

In any embodiment, the at least one dry component comprises an enzyme component. The enzyme component is capable of reacting with an enzyme substrate in a reaction that utilizes oxygen (e.g., dissolved oxygen in an aqueous medium). In use, an enzyme-mediated oxygen depletion system of the present disclosure comprises a first effective amount of the enzyme component and a second effective amount of the enzyme substrate component. When brought into fluid communication in an aqueous liquid in the growth region of a culture device according to the present disclosure, the first effective amount and the second effective amount are capable of reacting to reduce a first dissolved-oxygen concentration in the aqueous liquid to a second dissolved-oxygen concentration that is substantially lower than the first dissolved-oxygen concentration, the second dissolved oxygen concentration being sufficiently low enough to facilitate the growth of an anaerobic microorganism or a microaerophilic microorganism.

In any embodiment, the first effective amount and the second effective amount are selected such that reducing the first dissolved oxygen concentration to the second dissolved oxygen concentration occurs within about 120 minutes after bringing the enzyme component and enzyme substrate component into fluidic contact in predefined volume of aqueous liquid in the growth region of the culture device. In any embodiment, the first effective amount and the second effective amount are selected such that reducing the first dissolved oxygen concentration to the second dissolved oxygen concentration occurs within about 60 minutes after bringing the enzyme component and enzyme substrate component into fluidic contact in predefined volume of aqueous liquid in the growth region of the culture device. In any embodiment, the first effective amount and the second effective amount area selected such that reducing the first dissolved oxygen concentration to the second dissolved oxygen concentration occurs within about 30 minutes after bringing the enzyme component and enzyme substrate component into fluidic contact in predefined volume of aqueous liquid in the growth region of the culture device.

A person having ordinary skill in the art recognizes that, within a temperature range that may be specific to the particular enzyme or reaction, the rate of many enzyme-catalyzed reactions is generally related to the temperature of the reaction. In any embodiment, reducing the first dissolved oxygen concentration to the second dissolved oxygen concentration occurs at a temperature between ambient temperature (e.g., about 23 degrees C) and about 42 degrees C, inclusive. Thus, in any embodiment of a method according to the present disclosure, it is not required to incubate the culture device at an elevated temperature (i.e., above ambient temperature) in order to reduce the first dissolved oxygen concentration to the second dissolved oxygen concentration occurs within about 120 minutes, about 60 minutes, or about 30 minutes after bringing the enzyme component and enzyme substrate component into fluidic contact in predefined volume of aqueous liquid in the growth region of the culture device.

A number of oxidoreductase enzyme-mediated, oxygen-consuming reactions are known including, for example, reactions catalyzed by peroxidase, glucose oxidase, laccase, tyrosinase, and ascorbic acid oxidase. However, some of the oxidoreductase enzymes (e.g., peroxidase) react with enzyme substrates (e.g., hydrogen peroxide) that are relatively unstable and are toxic to microorganisms (e.g., in particular, to some anaerobic microorganisms) at relatively low aqueous concentrations. In addition, some of the oxidoreductase enzymes (e.g., glucose oxidase) react with oxygen to produce reaction products (e.g., hydrogen peroxide) that are toxic to microorganisms. In any embodiment, catalase or other peroxide-consuming enzymes can be added to the reaction in order to reduce the level of accumulated hydrogen peroxide. Therefore, a suitable enzyme-mediated oxygen depletion system according to the present disclosure utilizes an enzyme substrate that, when present in an effective amount in the growth region of the culture device, can be in fluidic communication with an anaerobic microorganism under conditions suitable for anaerobic microbial growth without substantially inhibiting growth of the anaerobic microorganism. Similarly, a suitable enzyme-mediated oxygen depletion system according to the present disclosure utilizes an enzyme and enzyme substrate that, when each are present in effective amounts in the growth region of the culture device and together consume enough oxygen to create a low-oxygen or anaerobic local environment, produce quantities and types of reaction products that can be in fluidic communication with an anaerobic microorganism under conditions suitable for anaerobic microbial growth without substantially inhibiting growth of the anaerobic microorganism.

A person having ordinary skill in the art will recognize the amount of oxygen removed from the growth region of a culture device of the present disclosure within a period of time suitable for culturing microorganisms is dependent *inter alia* upon the quantities (i.e., activity) of enzyme component and enzyme substrate component. The experimental results presented herein show the relationship between the quantity of enzyme substrate component available to participate in the oxygen-depleting reaction and the rate and extent of oxygen removal from the growth region. Therefore, by adjusting the amounts of the components of the enzyme-mediated oxygen depletion system according to the present disclosure, the culture device can be configured for culturing microaerotolerant microorganisms, for culturing microaerophilic microorganisms, or for culturing obligately anaerobic microorganisms.

Nonlimiting examples of suitable enzyme-mediated oxygen depletion systems according to the present disclosure include a system that comprises ascorbic acid oxidase and its corresponding enzyme substrate (e.g., L-ascorbic acid) and a system that comprises laccase and its corresponding enzyme substrate (e.g., *p*-diphenol or 2,6-dimethoxyphenol).

Returning to the drawings, in any embodiment, the culture device 10 optionally can comprise a second dry coating (shown in FIG. 2) disposed on the inner surface 20 of the second substrate 18. The second dry coating may comprise a dry, cold-water-soluble gelling agent and/or a component of an enzyme-mediated oxygen depletion system. In any embodiment, the component in the second coating comprises a first effective amount of a substantially dry enzyme component of the enzyme-mediated oxygen depletion system. Alternatively, in any embodiment, the component in the second coating comprises a second effective amount of a substantially dry enzyme substrate capable of reacting with the enzyme component in a reaction that utilizes oxygen. Suitable gelling agents for use in the second dry coating include those gelling agents described above as suitable for the first dry coating.

FIG. 2 shows another embodiment of a culture device 10' according to the present disclosure. Similar to the device 10 of FIG. 1, the culture device 10' comprises a waterproof first substrate 12, a waterproof second substrate 18, and a growth region 45 disposed between the first substrate 12 and second substrate 18. The first substrate 12 has an inner surface 14 and an outer surface 16 opposite the inner surface. The second substrate 18 has an inner surface 20 and an outer surface 22 opposite the inner surface. In any embodiment, the inner surface 14 of the first substrate 12 is disposed in facing relationship with the inner surface 20 of the second substrate 18. In addition, a first dry coating 30 is disposed on at least a portion 40 of the inner surface 14 of the first substrate 12. Optionally, an adhesive layer 32 is disposed between at least a portion of the first dry coating 30 and the first substrate 12.

The second substrate 18 can be free of any coating, or can be coated, e.g., on the inner surface 20 that faces the first substrate 12 as shown in FIG. 2; with a layer of pressure-sensitive adhesive (e.g., adhesive 37). In any embodiment, a portion of the layer of adhesive 37 may be used to facilitate sealing the second substrate 18 to at least a portion of the first substrate 12 or a coating disposed thereon (e.g., first dry coating 30). Additionally or alternatively, a portion of the layer of adhesive 37 can be coated with a second dry coating 35 as described herein.

Adhesive 37 disposed on the second substrate 18 can be the same as or different from the adhesive 32 disposed on the first substrate 12. In addition, the second dry coating 35 disposed on the second substrate 18 can be the same as or different from the first dry coating 30 disposed on the first substrate 12. Coatings on second substrate 18 can cover the entire surface facing the first substrate, but preferably cover at least the part of the inner surface 20 that defines at least a portion 41 of the growth region 45 of the culture device 10'. Such coated second substrates are particularly preferred when it is desired to provide a device with more gelling agent than can be incorporated in the first dry composition alone.

Preferably, when the second dry coating 35 consists primarily of dry powder or dry powder agglomerate, the second coating is disposed on an adhesive layer 37 that is disposed on at least a portion of the inner surface 20 of the second substrate 18. The second dry coating 35 can be deposited onto the second substrate 18 or onto the optional adhesive layer 37 using compounding processes, adhesive coating processes, and liquid-coating processes and/or dry-coating processes described, for example, in U.S. Patent Nos. 4,565,783; 5,089,413; and 5,232,838.

As regards the second dry coating 35, the coating optionally can comprise any nutrient or nutrient medium that is cold-water-reconstitutable, that does not substantially interfere with the cold-water gelling properties of the gelling agent, and that supports growth of an anaerobic microorganism. The particular nutrient or nutrients suitable for use in the culture device will depend on the microorganism to be grown in the device, and will be easily selected by those skilled in the art. Generally, such nutrients are cold-water soluble. In any embodiment, the second dry coating 35 further can comprise a selective agent (e.g., a nutrient, an antibiotic, and combinations thereof) that facilitates the growth of a particular anaerobic microorganism or group of microorganisms over another microorganism or group of microorganisms.

Culture device 10'further comprises a spacer means disposed between the first substrate 12 and the second substrate 18, in order to create a well that serves to both define a location and thickness of the growth region 45 of the culture device 10' and to confine an aqueous sample within the growth region 45. Spacer means are illustrated in FIG. 2 as spacer 46 defining a circular hole 48. The walls of hole 48 provide a well of predetermined size and shape and defines the thickness of the growth region 45 of the culture device 10. Spacer 46 should be thick enough to form a well of the desired volume, e.g., 1 mL, 2 mL, or 3 mL, depending on the size of the growth region and the size of sample to be placed in the culture device. In any embodiment, spacer 46 is made of closed cell polyethylene foam; but any material that is hydrophobic (non-wetting), inert to microorganisms, and sterilizable can be used. In any embodiment, the spacer means can be coupled (e.g., via a pressure-sensitive adhesive) directly to the first substrate or second substrate. Additionally or alternatively, the spacer means can be coupled (e.g., via a pressure-sensitive adhesive) indirectly to the first substrate or second substrate (e.g., the spacer means can be coupled to the first or second dry coating that is coated onto the first or second substrate, respectively).

In the illustrated embodiment of FIG. 2, the culture device 10' comprises a first dry coating 30 disposed in the growth region 45 on the inner surface 14 of the first substrate 12 and a second dry coating 35disposed in the growth region on the inner surface 20 of the second substrate 18. The first dry coating 30 comprises a first effective amount of a component (e.g., the enzyme component) of the enzyme-mediated oxygen depletion system and the second dry coating 35 comprises second effective amount of another component (e.g., a corresponding enzyme substrate for the enzyme component) of the enzyme-mediated oxygen depletion system. Thus, when an aqueous liquid (e.g., water, an aqueous buffer, an aqueous nutrient medium, a sample containing water) is introduced into the growth region of the culture device, the first effective amount and second effective amount are placed in fluid communication and the system reacts with oxygen present in the coatings and aqueous liquid, thereby consuming the oxygen in the growth region of the culture device and creating an environment to facilitate growth of microaerophilic or anaerobic microorganisms.

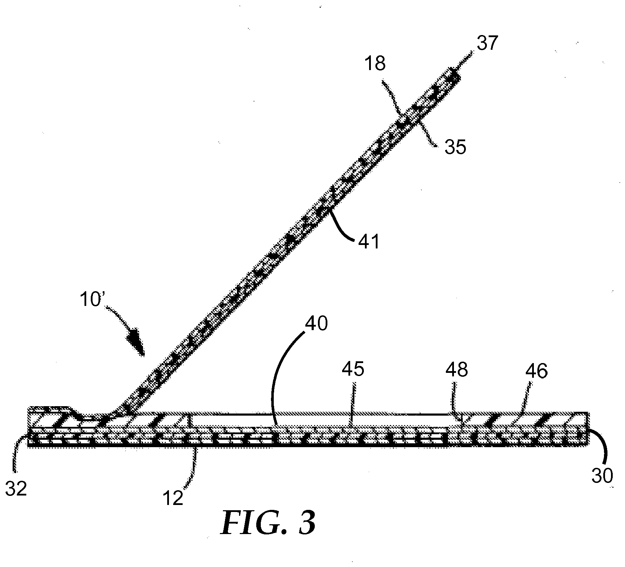

In the illustrated embodiment of FIGS. 2 and 3, the first dry coating 30 covers substantially all of the inner surface 14 of the first substrate 12, but only the portion 40 exposed by the circular hole 48 in the spacer 46 is accessible. Thus, the growth region 45 of culture device 10' is defined by the area of the circular hole 48 and the portions (41 and 40, respectively) of the first dry coating 30 and second dry coating 35 that overlap the circular hole 48 when the first substrate 12 and second substrate 18 are positioned adjacent each other. In this embodiment, the growth region is located away from the peripheral edges 50 and the communication between the growth region 45 and the external environment (e.g., air) is substantially blocked by the spacer 46.

Optionally, a culture device of the present disclosure further comprises a means for indicating oxygen in a culture device. Preferably, the means is capable of indicating a quantity (e.g., either a predetermined threshold quantity or a relative quantity) of oxygen present in the device. Advantageously, the means can indicate whether or when the enzyme-mediated oxygen depletion system has suitably depleted the oxygen in the growth region of the culture device to a concentration that facilitates the growth of microaerophilic or anaerobic microorganisms. Means for detecting oxygen in a culture device are known in the art and include, for example, redox dyes (e.g., methylene blue) and oxygen-quenched fluorescent dyes.

The means can be a luminescent compound that indicates the absence of oxygen inside of the device. Suitable oxygen indicators are disclosed in U.S. Patent No. 6,689,438 (Kennedy et al.). Luminescent compounds appropriate as indicators for a culture device of the present disclosure will display luminescence that is quenched by oxygen. More precisely, the indicators will luminesce upon exposure to their excitation frequency with an emission that is inversely proportional to the oxygen concentration. The indicator may be coated, laminated, or extruded onto another layer, or portion of another layer, within the device. Such a layer may be disposed in the growth region and optionally, is separated from the growth region by one or more other oxygen permeable layers. Suitable compounds for indicating oxygen include metallo derivatives of octaethylporphyrin, tetraphenylporphyrin, tetrabenzoporphyrin, the chlorins, or bacteriochlorins. Other suitable compounds include palladium coproporphyrin (PdCPP), platinum and palladium octaethylporphyrin (PtOEP, PdOEP), platinum and palladium tetraphenylporphyrin (PtTPP, PdTPP), camphorquinone (CQ), and xanthene type dyes such as erythrosin B (EB). Other suitable compounds include ruthenium, osmium and iridium complexes with ligands such as 2,2'-bipyridine, 1,10-phenanthroline, 4,7-diphenyl-1,10-phenanthroline and the like. Suitable examples of these include, tris(4,7,-diphenyl-1,10-phenanthroline)ruthenium(II) perchlorate, tris(2,2'-bipyridine)ruthenium(II) perchlorate, tris(1,10-phenanthroline)ruthenium(II) perchlorate, and the like.

A person having ordinary skill in the art will recognize that the rate of enzyme-catalyzed reactions may be affected by the pH of the environment in which the reaction takes place. Furthermore, a person having ordinary skill in the art will recognize that growth of a microaerophilic microorganism or an anaerobic microorganism in a culture device of the present disclosure may also be affected by the pH of the environment in which the microorganism is cultured. It is now known that the pH of the growth region can be preselected to facilitate the oxygen-consuming enzyme reaction while also facilitating the growth of microorganisms. In any embodiment, the pH of the growth region can be controlled by incorporating a buffering agent into at least one of the dry coatings and/or by inoculating the plate with a buffered aqueous solution.

In use, the growth region of a device of the present disclosure comprises a first effective amount of one component (e.g., the enzyme component) of the enzyme-mediated oxygen depletion system and a second effective amount of another component (e.g., a corresponding enzyme substrate for the enzyme component) of the enzyme-mediated oxygen depletion system in fluidic communication with one another. Typically, the maximum capacity for oxygen depletion by the system is determined by the amount of enzyme substrate in the growth region. Preferably, there is a sufficient quantity of enzyme substrate to react with substantially all of the dissolved oxygen in the hydrated growth region. More preferably, there is a sufficient quantity of enzyme substrate to react with more dissolved oxygen than would typically be found in the hydrated growth region.

In any embodiment, the growth region can be dimensioned to be hydrated with a 1 milliliter aqueous liquid volume. Water comprises about 0.54 µ moles of dissolved oxygen per milliliter. Thus, the first dry coating and/or second dry coating preferably comprises at least enough enzyme substrate to consume 0.54 µ moles of oxygen in a period of 120 minutes or less at about 22 degrees C to about 42 degrees C. More preferably, the first dry coating and/or second dry coating preferably comprises at least enough enzyme substrate to consume more than 0.54 µ moles of oxygen in a period of 120 minutes or less at about 22 degrees C to about 42 degrees C.

In any embodiment, the first dry coating and/or second dry coating can include any number of other components, such as dyes (e.g., a pH indicator), crosslinking agents, reagents (e.g., selective reagents or indicator reagents such as chromogenic or fluorogenic enzyme substrates), or a combination of any two or more of the foregoing components. For example, for some uses it is desirable to incorporate an indicator of microbial growth (e.g., a pH indicator, a chromogenic enzyme substrate, a redox dye) in the first and/or second dry coating or in an adhesive of to which the dry coating is adhered. Suitable dyes include those that are metabolized by or otherwise react with the growing microorganisms, and in so doing cause the colonies to be colored or fluorescent for easier visualization. Such dyes include triphenyl tetrazolium chloride, p-tolyl tetrazolium red, tetrazolium violet, veratryl tetrazolium blue and related dyes, and 5-bromo-4-chloroindolyl phosphate disodium salt. Other suitable dyes include those sensitive to pH changes during the growth of microorganisms, such as neutral red.

For some uses it is desirable to form a dry coating that, when reconstituted with an aqueous liquid, forms a hydrogel that is stiff enough to allow inoculation by streaking. To form streakable medium, an effective amount of a suitable cross-linking agent can be incorporated into one or more dry coating that includes a gelling agent. Suitable cross-linking agents do not substantially affect the growth of the intended microorganisms. Suitable types and amounts of cross-linking agents are easily selected by those skilled in the art. For example, with guar gum, cross-linking agents such as potassium tetraborate, aluminum salts, or calcium salts are suitable, and can be added in effective amounts, e.g., less than about 1.0 percent by weight of dry coating.

At least one dry coating can optionally include reagents necessary for carrying out certain microbiological tests. For example, antibiotics can be included for carrying out antibiotic susceptibility tests. For microorganism identification, differential reagents that undergo a color change in the presence of a particular type of microorganism can be included.

A culture device of the present can be prepared using a variety of techniques. Generally, a device can be made by hand or with common laboratory equipment as described herein and in U.S. Patent Nos. 4,565,783; 5,089,413; and 5,232,838, for example.

A first dry coating and/or second dry coating of a culture device of the present disclosure can comprise an adhered powder medium. The adhered powder medium is prepared and fixed by first forming a layer (layer 32 or layer 37, respectively) of adhesive on at least a portion of the growth region of the inner surface of the first and/or second substrate. The adhesive is preferably pressure-sensitive, insoluble in water, and substantially non-inhibitory to the growth of the microorganisms to be cultured in the device. Preferably, adhesive layer 32 or layer 37 is also sufficiently transparent when wet to enable viewing of microbial colonies.

A nonlimiting example of a suitable pressure-sensitive adhesive is a copolymer of 2-methylbutylacrylate/acrylic acid in a mole ratio of 90/10. Other preferred pressure sensitive adhesives that can be used include isooctylacrylate/acrylic acid in a mole ratio of 95/5 or 94/6 and silicone rubber. Adhesives that turn milky (e.g., opaque) upon exposure to water are less preferred, but can be used in conjunction with a non-transparent first substrate or in situations where colony visualization is not required. Heat-activated adhesives having a lower melting substance coated onto a higher melting substance and/or water-activated adhesives such as mucilage are also known and can be used in this invention. When incorporating an indicator reagent as described above in order to facilitate visualization of colonies, it is generally preferred to incorporate the indicator reagent in the adhesive or broth coating mixture, rather than in the powder.

The adhesive is coated (e.g., using a knife coater) onto the top surface of first substrate or second substrate to form an adhesive layer at a thickness that is preferably less than the average diameter of the particles of dry powder or agglomerated powder to be adhered to the adhesive. Generally, enough adhesive is coated in order to adhere the particles to the substrate (e.g., the first or second substrate described herein) but not so much that the particles become completely embedded in the adhesive. Generally, an adhesive layer about 5 µm to about 12 µm thick is suitable.

In order to form an adhered powder medium, a layer of cold-water-soluble powder (e.g., comprising a cold-water-soluble gelling agent) and; optionally; a nutrient, selective reagent, a component of the enzyme-mediated oxygen depletion system, or a combination of any two or more of the foregoing powders is then adhered substantially uniformly to the adhesive layer that is to be disposed in at least a portion of the growth area of the culture device.

Preferably, when gelling agent is included in the first dry coating and/or second dry coating, it is included in an amount such that a predetermined quantity of water or an aqueous sample, e.g., 1 to 3 ml, placed in the growth region will form a hydrogel having a suitable viscosity, e.g., about 1500 cps or more when measured at 60 rpm with a Brookfield Model L VF viscometer at 25° C. Hydrogels of this viscosity allow convenient handling and stacking of the culture devices during incubation and provide for distinct colony formation in the medium. For instance, 0.025 g to 0.050 g of powdered guar gum spread substantially uniformly over a surface area of 20.3 cm² will provide a sufficiently viscous medium when reconstituted with 1 to 3 ml of an aqueous sample. The size of the powder particles can be used to control the coating weight per unit area. For example, under conditions where a 100 mesh guar gum coats to a weight of about 0.05 g/20.3 cm², a 400 mesh guar gum coats to a weight of about 0.025 g/20.3 cm².

In any embodiment, the first dry coating or second dry coating can comprise one or more nutrients to facilitate growth of microorganisms. When the coating consists essentially of powders or powder agglomerates, the preferred ratio of gelling agent to nutrient in an adhered powder medium is determined by the particular microorganism to be grown on the device. For general purposes, however, a ratio from about 4 to 1 to about 5 to 1 (total gelling agent to total nutrient, based on weight) is preferred. The powder in an adhered powder medium can be applied to the adhesive layer (e.g., adhesive layer 32 and/or adhesive layer 37) by any means suitable for the application of a substantially uniform layer. Examples of suitable methods to apply the layer of powders include the use of a shaker-type device, or the use of a powder coater.

Another preferred method of forming a dry coating, i.e., first dry coating and/or second dry coating, is a liquid coating method. In this method, a liquid mixture (e.g., an aqueous liquid mixture) comprising the gelling agent, a component (e.g., the enzyme component) of the enzyme-mediated oxygen depletion system, and, optionally, a nutrient, selective agent, and/or indicator reagent is prepared. The liquid mixture is coated onto the first substrate or second substrate (e.g., using a knife coater) and, subsequently, substantially all of the liquid is removed (e.g., by flash evaporation) to produce the substantially dry (e.g., substantially water-free) coating. The gelling agent can serve to thicken the liquid mixture in order to facilitate its coating onto the substrate. For practical purposes, the amount of gelling agent is preferably less than that which will cause the mixture to thicken to the point where it is not practical to coat the medium onto the·substrate. These coated mixtures can be generally self-adherent to the substrate and, thus, do not require a layer of adhesive between the substrate and the medium.

In any embodiment, the first dry coating and the second dry coating may be prepared by a similar process (i.e., both prepared by a powder-coating process or by a liquid-coating process). Alternatively, in any embodiment, one of the dry coatings (e.g., the first dry coating) is coated using a liquid-coating process and another of the dry coatings (e.g., the second dry coating) is coated using a powder-coating process. In addition, the first dry coating and second dry coating may comprise the same constituents or different constituents.

In any embodiment, a culture device of the present disclosure comprises one substrate (e.g., the first substrate), having a dry coating (e.g., the first dry coating) that comprises a gelling agent and the enzyme component of an enzyme-mediated oxygen depletion system, and another substrate (e.g., the second substrate) having a dry coating (e.g., the second dry coating) that comprises a gelling agent and/or the enzyme substrate component of the enzyme-mediated oxygen depletion system. Preferably, the first dry coating is coated using a liquid-coating process and the second dry coating is coated using a powder-coating process.

When using culture device of the present disclosure, an accurate count of the colonies of microorganisms present can be desirable. Thus, in any embodiment, a culture device of the present disclosure may comprise a grid pattern on first substrate or, alternatively, on the second substrate. The grid pattern may comprise a square grid pattern such as, for example, the square grid pattern disclosed in U.S. Patent No. 4,565,783. The grid pattern may be produced on the first or second substrate by any suitable process such as printing methods, for example.

It is also contemplated that the enzyme-mediated oxygen depletion system can be used in a culture medium similar to that described in U.S. Patent No. 6,331,429. The culture medium, when disposed in a culture device as disclosed herein or, alternatively, when disposed between suitable first and second substrates as described herein, can be used in a method of culturing microaerophilic or obligately-anaerobic microorganisms.

In another aspect, the present disclosure provides a method of making a self-contained anaerobic environment-generating culture device. The method comprises depositing a first coating onto a portion of a first substrate, the first coating being formed using a liquid mixture comprising a liquid and an effective amount of an enzyme component of an enzyme-mediated oxygen depletion system; drying the coating; and positioning the first substrate adjacent the second substrate such that the first coating faces the second coating and such that a growth region disposed between the first substrate and the second substrate overlaps a portion of the first coating and a portion of the second coating.

The enzyme component of the first coating is an enzyme capable of reacting with an enzyme substrate and oxygen to form a product. The reaction occurs in an aqueous mixture and results in localized depletion of oxygen from the aqueous mixture, as described herein. Optionally, in any embodiment of the method, an adhesive layer may be disposed on the first substrate between the first coating and the first substrate. Optionally, in any embodiment of the method, the first coating may further comprise a nutrient or nutrient medium, a selective agent, an indicator reagent, a crosslinking agent, a dye or a combination of any two or more of the foregoing, as described herein. The composition of the first coating is adjusted so that an effective amount of enzyme component (e.g., ascorbic acid oxidase) is distributed (e.g., uniformly distributed) across a first portion of a growth region that is defined by the first substrate.

In any embodiment of the method, optionally, the method further comprises forming a second coating onto a second substrate. The second coating comprises an enzyme substrate component of the enzyme-mediated oxygen depletion system and is capable, in an aqueous medium, of reacting with the enzyme component and oxygen to form a product. Optionally, in any embodiment of the method, an adhesive layer may be disposed on the second substrate between the second coating and the second substrate. In any embodiment, the second coating comprises substantially dry powder or a powder agglomerate disposed on an adhesive layer adhered to the second substrate. The composition of the second coating is adjusted so that an effective amount of enzyme substrate component (e.g., sodium ascorbate) is distributed (e.g., uniformly distributed) across a second portion of a growth region that is defined by the second substrate.

In any embodiment of the method, the liquid used to form the liquid mixture comprises an aqueous liquid. In any embodiment, the liquid used to form the liquid mixture comprises an organic liquid. In any embodiment of the method, the liquid mixture used to form the first coating further comprises a cold-water-soluble gelling agent.

In any embodiment of the method, depositing a first coating onto a portion of a first substrate comprises mixing the enzyme component and the gelling agent in a predetermined volume of the aqueous liquid to form a coating mixture and coating the coating mixture onto the first substrate. In any embodiment, coating the coating mixture onto the first substrate can comprise coating the coating mixture onto an adhesive layer disposed on the first substrate, as described herein.

Drying the first coating can be performed by a number of processes known in the art. The coating can be dried in an oven (e.g., a gravity oven, a convection oven), for example, according to the process described in U.S. Patent No. 5,601,998. Preferably, the first coating is dried until it is substantially water-free. As used herein, the phrases "substantially dry", "substantially water-free" or the like refer to a coating which has a water content no greater than about the water content of the dehydrated coating once it has been permitted to equilibrate with the ambient environment.

Positioning the first substrate adjacent the second substrate, such that the first coating faces the second coating and such that a growth region disposed between the first substrate and the second substrate overlaps a portion of the first coating and a portion of the second coating, can be performed in a variety of ways. Representative examples of suitable methods of positioning the first and second substrates adjacent each other so that portions of the first and second coatings overlap one another are shown in FIGS. 1-3. It can be seen that the overlapping configuration permits an operator to deposit an aqueous liquid between the first and second substrates thereby placing the first coating and second coating (if present) into fluid communication.

In yet another aspect, the present disclosure provides a culture device for enumerating Clostridium difficile colony-forming units in a sample. The culture device comprises a first substrate having opposing inner and outer surfaces, a second substrate having opposing inner and outer surfaces, and a growth region disposed therebetween as described hereinabove. The culture device further comprises a first effective amount of a substantially dry enzyme component of an enzyme-mediated oxygen depletion system, the first effective amount disposed in a first coating the growth region; a second effective amount of a substantially dry enzyme substrate component of the enzyme-mediated oxygen depletion system, the second effective amount disposed in a second coating in the growth region; and an effective amount of a substantially dry nutrient composition disposed in the growth region. In any embodiment, the nutrient composition comprises a nutrient mixture (e.g., brain heart infusion and/or yeast extract) to facilitate growth of a fastidious microorganism. In any embodiment, the culture device further comprises an effective amount of at least one substantially dry selective agent (e.g., a bile salt such as sodium taurocholate, for example; one or more antibiotic such as Cefoxitin and Cycloserine, for example) that, when hydrated with a predetermined volume of aqueous liquid, substantially permits growth of *C. difficile* and substantially inhibits growth of non- *C. difficile* microorganisms such as, for example, *E. coli, S. aureus, C. sporogenes, C. perfringens, Bacteroides fragilis, Prevotella melaninogencia, Fusobacterium* species, and *Peptostreptococcus anaerobius.* Optionally, the culture device further comprises an effective amount of a substantially dry reducing agent (e.g., sodium thioglycollate and/or L-cysteine) disposed in the growth region.

In yet another aspect, the present disclosure provides a method of detecting a microorganism. In any embodiment, the method uses a self-contained anaerobic environment-generating culture device of the present disclosure. The culture device comprises a first substrate having opposing inner and outer surfaces; a second substrate having opposing inner and outer surfaces; a growth region disposed between the inner surfaces of the first and second substrates; an effective amount of a substantially dry enzyme component of an enzyme-mediated oxygen depletion system, the first effective amount disposed in the growth region; and a substantially-dry, cold-water-soluble gelling agent disposed in the growth region; wherein the first substrate and second substrate are substantially nontransmissible to gaseous oxygen. Optionally, the culture device further comprises an effective amount of a substantially dry enzyme substrate component of the enzyme-mediated oxygen depletion system, the second effective amount disposed in the growth region. In a preferred embodiment, the culture device further comprises a spacer that defines a perimeter of the growth region.

In any embodiment of the method, the substantially-dry, cold-water-soluble gelling agent disposed in the growth region is of the self-contained anaerobic environment-generating culture device is hydrated with a predetermined volume of aqueous liquid in order to i) facilitate a reaction between the components of the enzyme-mediated oxygen depletion system and ii) provide an aqueous environment to facilitate growth of microorganisms. The cold-water-soluble gelling agent in the culture device can be hydrated before the sample is deposited into the device, while the sample is deposited into the device, and/or after the sample is deposited into the device, as discussed herein.

In any embodiment, the predetermined volume of aqueous liquid used to hydrate and/or inoculate the culture device is about 1 milliliter. In any embodiment, the predetermined volume of aqueous liquid used to hydrate and/or inoculate the culture device is about 2 milliliters. In any embodiment, the predetermined volume of aqueous liquid used to hydrate and/or inoculate the culture device is about 3 milliliters. In any embodiment, the predetermined volume of aqueous liquid used to hydrate and/or inoculate the culture device is about 4 milliliters. In any embodiment, the predetermined volume of aqueous liquid used to hydrate and/or inoculate the culture device is about 5 milliliters. In any embodiment, the predetermined volume of aqueous liquid used to hydrate and/or inoculate the culture device is about 10 milliliters. In any embodiment, the aqueous liquid used to hydrate the growth region of the culture device is distributed over an area that results in approximately one milliliter of liquid per 20.3 cm² of growth region.

In any embodiment, the effective amount of enzyme substrate component (e.g., L-ascorbic acid or a salt thereof) in the growth region of the culture device can be selected based upon the predetermined volume of aqueous liquid that is to be deposited in the growth region. For example, if the predetermined volume is about 1 milliliter, the effective amount of enzyme substrate component (e.g., L-ascorbic acid or a salt thereof) disposed in or deposited into the growth region can be about 3 micromoles to about 4.5 micromoles (to generate an environment for culturing microaerophilic or microaerotolerant microorganisms) 7 micromoles to about 30 micromoles (to generate an environment for culturing obligately-anaerobic microorganisms). For example, if the predetermined volume is about 2 milliliter, the effective amount of enzyme substrate component (e.g., L-ascorbic acid or a salt thereof) disposed in or deposited into the growth region can be about 6 micromoles to about 9 micromoles (to generate an environment for culturing microaerophilic or microaerotolerant microorganisms) 14 micromoles to about 60 micromoles (to generate an environment for culturing obligately-anaerobic microorganisms). A person having ordinary skill in the art will recognize the number of micromoles of enzyme substrate for a given enzyme-mediated oxygen depletion system may vary depending upon how many micromoles of oxygen are consumed per micromole of enzyme substrate component in the reaction catalyzed by the particular enzyme component.

In any embodiment, the predetermined volume of aqueous liquid is distributed in a growth region having a predetermined area (e.g., the area defined by the hole 48 in the spacer 46 of the culture device 10' of FIG. 2). Thus, when coating the enzyme substrate component onto the first or second substrate, the effective amount of enzyme substrate component (e.g., L-ascorbic acid or a salt thereof) in the growth region of the culture device can be selected based upon the predetermined area of the growth region. For example, if the predetermined area of the growth region is defined by a circle having a diameter of about 2 inches (about 5.1 cm), the effective amount of enzyme substrate component (e.g., L-ascorbic acid or a salt thereof) disposed in or deposited into the growth region can be about 0.15 micromoles/cm² to about 0.22 micromoles/cm² (to generate an environment for culturing microaerophilic or microaerotolerant microorganisms when the growth region is hydrated with 1 mL of liquid) 0.35 micromoles/cm² to about 1.5 micromoles/cm² (to generate an environment for culturing obligately-anaerobic microorganisms when the growth region is hydrated with 1 mL of liquid).

Typically, the culture device is placed on a generally-level surface and the first substrate and second substrate are separated (e.g., the upper substrate is lifted) to provide access to the growth region of the culture device while the growth region is being hydrated and/or inoculated. Advantageously, the culture device can be hydrated and/or inoculated in an aerobic environment (i.e., in air). Typically, the aqueous liquid (which may include sample material to be tested) used to hydrate the device is pipetted onto the growth region between the first and second substrates. After the predefined volume of aqueous liquid is deposited into the growth region, the culture device is closed. Optionally, a flat or concave spreader, similar to those used to inoculate PETRIFILM culture devices, can be used to distribute the aqueous liquid over a predefined area within the culture device.

After the culture device is hydrated, if the enzyme component and the enzyme substrate component of the enzyme-mediated oxygen depletion system are present in the growth region (e.g., the enzyme component is present in a substantially dry first composition in the growth region on one of the substrates and the enzyme substrate component is present in a substantially dry second composition in the growth region on the other substrate, hydrating the growth region comprises placing the enzyme component in aqueous fluid communication with the enzyme substrate component.

When placed in fluidic communication; the first composition, second composition, and aqueous liquid form a mixture that comprises a first concentration of dissolved oxygen. In any embodiment, the first concentration of dissolved oxygen may be a concentration that substantially inhibits growth of an obligately-anaerobic microorganism, a microaerophilic microorganism, and/or a microaerotolerant microorganism. In these embodiments, placing the components in aqueous fluid communication initiates the reaction of the enzyme with the enzyme substrate and any oxygen dissolved in the coatings and/or aqueous fluid, thereby reducing the first concentration of dissolved oxygen in the growth region to a second concentration that is lower than the first concentration (e.g., at least about 50% lower, at least about 60% lower, at least about 70% lower, at least about 80%, at least about 90%, at least about 95%, at least about 98%, at least about 99% lower than the first concentration).

In any embodiment, reducing the first concentration of dissolved oxygen to the second concentration of dissolved oxygen can comprise reducing the dissolved oxygen to a second concentration that is low enough to support the growth of a microaerotolerant microorganism. In any embodiment, reducing the first concentration of dissolved oxygen to the second concentration of dissolved oxygen can comprise reducing the dissolved oxygen to a second concentration that is low enough to support the growth of a microaerophilic microorganism. In any embodiment, reducing the first concentration of dissolved oxygen to the second concentration of dissolved oxygen can comprise reducing the dissolved oxygen to a second concentration that is low enough to support the growth of an obligately-anaerobic microorganism.

In any embodiment, reducing the first concentration of dissolved oxygen to the second concentration of dissolved oxygen comprises reducing the dissolved oxygen to the second concentration in less than or equal to about 120 minutes after the mixture is formed. In any embodiment, reducing the first concentration of dissolved oxygen to the second concentration of dissolved oxygen comprises reducing the dissolved oxygen to the second concentration in less than or equal to about 90 minutes after the mixture is formed. In any embodiment, reducing the first concentration of dissolved oxygen to the second concentration of dissolved oxygen comprises reducing the dissolved oxygen to the second concentration in less than or equal to about 60 minutes after the mixture is formed. In any embodiment, reducing the first concentration of dissolved oxygen to the second concentration of dissolved oxygen comprises reducing the dissolved oxygen to the second concentration in less than or equal to about 45 minutes after the mixture is formed. In any embodiment, reducing the first concentration of dissolved oxygen to the second concentration of dissolved oxygen comprises reducing the dissolved oxygen to the second concentration in less than or equal to about 30 minutes after the mixture is formed.

In any embodiment, the aqueous liquid used to hydrate the culture device comprises water (e.g., sterile, deionized water; biological buffers; sterile nutrient medium) and, optionally, may comprise one or more additive. The one or more additive may perform a variety of functions in the method. For example, in any embodiment, the one or more additive may comprise a nutrient or a nutrient medium to support growth of an anaerobic, microaerophilic, or microaerotolerant microorganism to be cultured in the device. Such nutrients or nutrient media are well known in the art and may be selected based upon the particular microorganism to be cultured. The nutrient or nutrient medium should not substantially interfere with the enzyme-mediated oxygen depletion system. This can be tested readily by using an oxygen sensor as described herein.

Alternatively or additionally, in any embodiment, the additive comprises at least a portion (or all) of the effective amount of the substantially dry enzyme substrate component (e.g., L-ascorbic acid, metal salts of L-ascorbic acid, hydroquinones such as 2,5-dichlorohydroquinone, D-erythroascorbic acid, chlorohydroquinone, for example) of the enzyme-mediated oxygen depletion system. Thus, the culture device can comprise an effective amount of the substantially dry enzyme component of the enzyme-mediated oxygen depletion system and not comprise an effective amount of the enzyme substrate component of the enzyme-mediated oxygen depletion system. In these embodiments, the enzyme substrate component is an additive in the aqueous liquid used to hydrate the culture device. Thus, depositing the aqueous liquid into the growth region bring the substantially dry enzyme component present in the culture device into aqueous fluidic communication with the enzyme substrate component present in the aqueous fluid, thereby initiating the reaction of the enzyme with the enzyme substrate and any oxygen dissolved in the coatings and/or aqueous fluid. This reaction depletes the oxygen from the growth region of the culture device.

Alternatively or additionally, in any embodiment, the additive comprises one or more selective agent (e.g., an antibiotic, a salt) that favors growth of one microorganism over at least one other microorganism. In an embodiment, the selective agent favors the growth of *Clostridium difficile.* Alternatively or additionally, in any embodiment, the additive comprises an indicator reagent (e.g., a pH indicator, a redox indicator, a chromogenic enzyme substrate, a fluorogenic enzyme substrate) for detecting the presence of a microorganism. A person having ordinary skill in the art will recognize selective agents and indicator reagents useful for detecting a microorganism. The selective agent and/or indicator should not substantially interfere with the enzyme-mediated oxygen depletion system. This can be tested readily by using an oxygen sensor as described herein. Advantageously, by adding the one or more selective agent and/or indicator to a solution that is used to hydrate the culture device, a particular culture device that includes a particular nutrient medium (e.g., a general nutrient medium for growing a wide variety of microorganisms) can be used for culturing a wide variety of specific microorganisms or groups of microorganisms.

If the culture device is hydrated before sample material is placed into the device, the cold-water-soluble gelling agent optionally may be permitted to hydrate and form gel (e.g., at room temperature) for several minutes up to about 30 minutes or more before the device is reopened to inoculate with the culture material. During the period in which the gelling agent is permitted to hydrate and form a gel, the components of the enzyme-mediated oxygen depletion system, if all are present, reduce the concentration of dissolved oxygen in the hydrated gelling agent from a first concentration to a second concentration that facilitates growth of microaerotolerant, microaerophilic, facultatively-anaerobic, or obligately-anaerobic microorganisms, as discussed herein.

Before or after the growth region of the culture device is hydrated, sample material can be contacted with the growth region in a variety of ways that are known in the art. In any embodiment, the sample material is contacted with the growth area by depositing the sample material into the growth area. This can be done, for example, by pipetting, by contacting the growth region with a swab that was used to obtain the sample material (e.g., by swabbing a surface), by contacting the growth region with an inoculating loop or needle (e.g., using a streak-plate technique), or by placing a sample capture device (e.g., a swab, sponge, or membrane filter) directly into the growth region and re-closing the culture device. After the sample is deposited and the culture device is re-closed (taking care not to entrap macroscopically-visible air bubbles in the culture device), the enzyme-mediated oxygen depletion system resumes depletion of the dissolved oxygen in the growth region.

In any embodiment, after the growth region of the culture device is hydrated and the gelling agent forms a gel, the culture device can be used as a contact plate (e.g., a Rodac plate). Thus, after the gel has formed, the first and second substrates of the culture device are separated, exposing the hydrated gel in the growth region. The hydrated gel is contacted with a surface to be samples, and the culture device is re-closed, taking care not to entrap macroscopically-visible air bubbles in the culture device. Advantageously, the contact plate procedure can be performed in an aerobic environment and, after re-closing the culture device, a reduced-oxygen environment can be re-established in the culture device by the enzyme-mediated oxygen depletion system.

In any embodiment of the method, after the growth area is contacted (e.g., inoculated) with a sample and closed, the culture device is incubated for a period of time (e.g., a predetermined period of time). The incubation conditions (e.g., the incubation temperature) can affect the rate of growth of the microaerotolerant, microaerophilic, facultatively-anaerobic, or obligately-anaerobic bacteria and may affect the types of bacteria that are detected, as is well known by a person having ordinary skill in the art. For example, incubation at lower temperatures (e.g., about 25° C) can allow for the detection of psychrotrophic bacteria). Incubation at higher temperatures (e.g., about 30° C, about 32° C, about 35° C, about 37° C) may facilitate faster growth of certain microaerotolerant, microaerophilic, facultatively-anaerobic, or obligately-anaerobic microorganisms.

In some embodiments, the culture device can be incubated for at least about 16 hours, at least about 18 hours, at least about 24 hours, or at least about 48 hours. In some embodiments, the culture device can be incubated not more than about 24 hours, not more than about 48 hours, or not more than about 72 hours. In certain preferred embodiments, the culture device is incubated about 24 hours to about 48 hours. In any embodiment, the culture device can be incubated, and maintain a reduced-oxygen environment therein, for about 72 hours, for about 96 hours, for about 120 hours, for about 7 days, or for about 8 days before detecting or counting anaerobic microorganism colonies growing in the growth region. In any embodiment, incubating the culture device for a period of time sufficient to permit formation of a microbial colony comprises incubating the culture device for the period of time in an aerobic gaseous environment.

After the inoculated culture device is incubated, the method further comprises detecting a microbial colony. Microbial colonies can be detected in the culture device by a variety of techniques that are known in the art. After a suitable incubation period, the absence of a microorganism can be detected in a culture device by the absence of observable colonies, the absence of a change in a growth indicator (e.g., a pH indicator, a chromogenic enzyme substrate, a redox indicator such as TTC, a fluorogenic enzyme substrate) and the absence of gas bubbles associated with the metabolism of the fermentable carbohydrate in the growth medium.

An acid zone associated with a colony of microorganisms can be detected visually and/or by the use of an imaging system. For example, in a method wherein the culture medium comprises bromcresol purple as a pH indicator, the culture medium will have a purple or gray appearance at about a neutral pH. As the microorganisms grow and ferment a carbohydrate (e.g., glucose) in the culture medium, the bromcresol purple indicator will appear yellow adjacent the growing bacterial colonies. For example, in a method wherein the culture medium comprises chlorophenol red as a pH indicator, the culture medium will have a red or violet appearance at about a neutral pH. As the microorganisms and ferment a carbohydrate in the culture medium, the chlorophenol red indicator will appear yellow adjacent the growing microbial colonies.

Gas bubbles, if present in the growth region and associated with a colony of microorganisms, can be detected visually and/or by the use of an imaging system. The gas bubbles may be associated with a visible colony and/or an acid zone detectable by a change in the color of a pH indicator in a region adjacent the colony of microorganisms. The gas bubble may comprise carbon dioxide generated by anaerobic fermentation of a carbohydrate, for example. Some microorganisms may produce hydrogen sulfide (H₂S) gas via the reduction of sulfate ions. The hydrogen sulfide can react with metal ions (e.g., Fe ions) present in the culture device to produce an insoluble black precipitate (e.g., FeS) adjacent a sulfate-reducing bacterial colony. Thus, in any embodiment, a first coating or second coating of the present disclosure may comprise a metal salt capable of reacting with hydrogen sulfide to produce an insoluble, colored precipitate.

In any of the above embodiments of the method, the culture device may have a growth region that, prior the step of contacting the growth region with the predefined volume, does not comprise an effective amount of a substantially dry enzyme component of an enzyme-mediated oxygen depletion system. In these embodiments, the method further comprises a step of depositing an effective amount of an enzyme component of the enzyme-mediated oxygen depletion system into the growth region. The enzyme component can be dispensed in a dry form or in a liquid form. In any embodiment, the enzyme component can be mixed with a liquid sample prior to depositing the sample into the growth region. In any embodiment, the method further comprises depositing an effective amount of a substantially dry enzyme substrate component of the enzyme-mediated oxygen depletion system into the growth region.

In any of the above embodiments, the method further can comprise obtaining an image of the culture device. In these embodiments, detecting the presence or absence of a microorganism comprises displaying, printing, or analyzing the image of the culture device. The imaging system comprises an imaging device and may comprise a processor. In some embodiments, the imaging device can comprise a line-scanner or an area scanner (e.g., a camera). The imaging device can include a monochromatic (e.g., black-and-white) or a polychromatic (e.g., color) scanner. Advantageously, monochromatic imaging systems can provide higher resolution images, which may improve the accuracy of the result and/or reduce the time necessary to detect the presence of microorganisms in the culture device.

In some embodiments, the imaging system further comprises an illumination system. The illumination system may include at least one source of broad-spectrum visible light (e.g., a "white" light). In some embodiments, the illumination system may include at least one source of narrow-spectrum visible light (e.g., a light-emitting diode that emits a relatively narrow bandwidth of visible light such as, for example, red, green, or blue light). In certain embodiments, the illumination system may include a source of narrow-spectrum visible light (e.g., a light-emitting diode) with a light emission peak at a preselected wavelength (e.g., about 525 nm).

The image can be obtained from light reflected by the components (e.g., microbial colonies, growth media, and indicators) in the growth region of the culture device or the image can be obtained from light transmitted through the components in the growth region of the culture device. Suitable imaging systems and corresponding illumination systems are described, for example, in International Patent Publication No. WO 2005/024047 and U.S. Patent Application Publication Nos. US 2004/0101954 and US 2004/0102903. Non-limiting examples of suitable imaging systems include PETRIFILM Plate Reader (PPR), available from 3M Company (St. Paul, MN), the PETRISCAN Colony Counter available from Spiral Biotech (Norwood, MA), and PROTOCOL and ACOLYTE plate scanners available from Synbiosis (Cambridge, U.K.).

In some embodiments, obtaining an image comprises obtaining a wavelength-biased image. For example, the imaging system can include a bias filter that biases the light collected by the imaging device. Filter elements are known in the art and include both "cut-off" filters (i.e., filters that allow the passage of light wavelengths either above or below a certain specified wavelength) and "band-pass" filters (i.e., filters that allow the passage of light wavelengths between certain specified upper and lower limits). A bias filter can be positioned between the illumination source and the culture device. Alternatively or additionally, a bias filter can be positioned between the culture device and the imaging device.

### EXEMPLARY EMBODIMENTS

Embodiment A is a method of detecting a microorganism in a sample, the method comprising:
contacting a growth region of a self-contained anaerobic environment-generating culture device with a predefined volume of aqueous liquid wherein, prior the step of contacting the growth region with the predefined volume, the growth region comprises a dry, cold-water-soluble gelling agent;
depositing an effective amount of an enzyme component of an enzyme-mediated oxygen depletion system into the growth region;
contacting the growth region with a sample;
incubating the culture device for a period of time sufficient to permit formation of a microbial colony; and
detecting the microbial colony.

Embodiment B the method of Embodiment A, further comprising depositing an effective amount of an enzyme substrate component of the enzyme-mediated oxygen depletion system into the growth region.

Embodiment C is a method of detecting a microorganism in a sample, the method comprising:
contacting a growth region of a self-contained anaerobic environment-generating culture device with a predefined volume of aqueous liquid wherein, prior the step of contacting the growth region with the predefined volume, the growth region comprises a dry, cold-water-soluble gelling agent and an effective amount of a substantially dry enzyme component of an enzyme-mediated oxygen depletion system;
contacting the growth region with a sample;
incubating the culture device for a period of time sufficient to permit formation of a microbial colony; and
detecting the microbial colony.

Embodiment D is a method of detecting a microorganism in a sample, the method comprising:
contacting a growth region of a self-contained anaerobic environment-generating culture device with a predefined volume of aqueous liquid wherein, prior the step of contacting the growth region with the predefined volume, the growth region comprises a dry, cold-water-soluble gelling agent;
depositing an effective amount of an enzyme substrate component of an enzyme-mediated oxygen depletion system into the growth region;
contacting the growth region with a sample;
incubating the culture device for a period of time sufficient to permit formation of a microbial colony; and
detecting the microbial colony.

Embodiment E the method of Embodiment D, further comprising depositing an effective amount of an enzyme component of the enzyme-mediated oxygen depletion system into the growth region.

Embodiment F is the method of any one of Embodiments A through C, wherein the growth region of the culture device further comprises an effective amount of a substantially dry enzyme substrate component of the enzyme-mediated oxygen depletion system.

Embodiment G is the method of any one of the preceding Embodiments, wherein contacting the growth region with the aqueous liquid comprises contacting the growth region with the sample.

Embodiment H is the method of any one of the preceding Embodiments, wherein the step of contacting the growth region with the sample is not simultaneous with the step of contacting the growth region with the predefined volume of aqueous liquid.

Embodiment I is the method of Embodiment H, wherein the step of contacting the growth region with the sample occurs after the step of contacting the growth region with the predefined volume of aqueous liquid.

Embodiment J is the method of Embodiment H, wherein the step of contacting the growth region with the sample occurs before the step of contacting the growth region with the predefined volume of aqueous liquid.

Embodiment K is the method of any one of the preceding Embodiments, wherein contacting the growth region with the sample or contacting the growth region with the predefined volume of aqueous liquid comprises providing an additive in the sample or the predefined volume of aqueous liquid.

Embodiment L is the method of Embodiment K, wherein the additive comprises an effective amount of a selective agent or an indicator for detecting microbial growth.

Embodiment M is method of Embodiment L, wherein the effective amount of the selective agent substantially permits germination and growth of the *Clostridium difficile* microorganism in the culture device and the effective amount of selective agent substantially inhibits growth of *E. coli, S. aureus, C. sporogenes, C. perfringens, Bacteroides fragilis, Prevotella melaninogencia, Fusobacterium* species, and/or *Peptostreptococcus anaerobius.*

Embodiment N is the method of any one of the preceding Embodiments, wherein incubating the culture device for a period of time sufficient to permit formation of a microbial colony comprises incubating the culture device for the period of time in an aerobic gaseous environment.

Embodiment O is the method of any one of the preceding Embodiments, wherein a first dry composition is coated on a first substrate of the culture device and a second dry composition is coated onto a second substrate of the culture device.

Embodiment P is the method of any one of Embodiments A through O:
wherein, when the first composition, second composition, and the aqueous liquid are placed in fluidic communication in the growth region of the culture device, they form an aqueous mixture that comprises a first concentration of dissolved oxygen;
wherein, while the device is held in an aerobic environment for less than or equal to about 120 minutes after the liquid mixture is formed, the first concentration of dissolved oxygen is reduced by the enzyme-mediated oxygen depletion system to a second concentration of dissolved oxygen that does not substantially inhibit growth of a microaerotolerant microorganism.

Embodiment Q is the method of any one of Embodiments A through O:
wherein, when the first composition, second composition, and the aqueous liquid are placed in fluidic communication in the growth region of the culture device, they form an aqueous mixture that comprises a first concentration of dissolved oxygen;
wherein, while the device is held in an aerobic environment for less than or equal to about 120 minutes after the liquid mixture is formed, the first concentration of dissolved oxygen is reduced by the enzyme-mediated oxygen depletion system to a second concentration of dissolved oxygen that does not substantially inhibit growth of a microaerophilic microorganism.

Embodiment R is the method of any one of Embodiments A through O:
wherein, when the first composition, second composition, and the aqueous liquid are placed in fluidic communication in the growth region of the culture device, they form an aqueous mixture that comprises a first concentration of dissolved oxygen;
wherein, while the device is held in an aerobic environment for less than or equal to about 120 minutes after the liquid mixture is formed, the first concentration of dissolved oxygen is reduced by the enzyme-mediated oxygen depletion system to a second concentration of dissolved oxygen that does not substantially inhibit growth of an obligately-anaerobic microorganism.

Embodiment S is the method of any one of the preceding Embodiments, wherein detecting the microbial colony comprises detecting a colony of *Campylobacter* microorganisms.

Embodiment T is the method of any one of Embodiments A though R, wherein detecting the microbial colony comprises detecting a colony of microorganisms that ferment glucose to lactic acid.

Embodiment U is a culture device for enumerating colonies of microorganisms, the device comprising:
a first substrate having opposing inner and outer surfaces;
a second substrate having opposing inner and outer surfaces;
a growth region disposed between the inner surfaces of the first and second substrates;
a first effective amount of a substantially dry enzyme component of an enzyme-mediated oxygen depletion system, the first effective amount disposed in a first coating the growth region;
a second effective amount of a substantially dry enzyme substrate component of the enzyme-mediated oxygen depletion system, the second effective amount disposed in a second coating in the growth region; and
a dry, cold-water-soluble gelling agent disposed in the growth region;
wherein the first substrate and second substrate are substantially nontransmissible to gaseous oxygen.

Embodiment V is the culture device of Embodiment U wherein, when the effective amounts of the enzyme component and the enzyme substrate component are brought into fluid communication in the growth region using a predefined volume of aqueous liquid, the enzyme component and the enzyme substrate component are capable of reacting to reduce a first dissolved-oxygen concentration in the aqueous liquid to a second dissolved-oxygen concentration that is substantially lower than the first dissolved-oxygen concentration.

Embodiment W is the culture device of Embodiment V, wherein the effective amounts of the enzyme component and the enzyme substrate component are selected such that reducing the first dissolved oxygen concentration to the second dissolved oxygen concentration occurs within 60 minutes of bringing the first effective amount and second effective amount into fluid communication in the predefined volume of aqueous liquid.

Embodiment X is the culture device of any one of Embodiments S through U, further comprising a spacer disposed between the first substrate and the second substrate, wherein the spacer defines a perimeter of the growth region in the culture device.

Embodiment Y is the culture device of any one of Embodiments U through X, further comprising a nutrient that facilitates growth of a microorganism.

Embodiment Z is the culture device of Embodiment Y, wherein the nutrient facilitates growth of a Clostridium difficile microorganism.

Embodiment AA is the culture device of Embodiment Z, further comprising an effective amount of a selective agent wherein, when the growth region is reconstituted with a predefined volume of aqueous liquid, the effective amount of selective agent substantially permits germination and growth of the *Clostridium difficile* microorganism in the culture device and the effective amount of selective agent substantially inhibits growth of *E. coli, S. aureus, C. sporogenes, C. perfringens, Bacteroides fragilis, Prevotella melaninogencia, Fusobacterium* species, and/or *Peptostreptococcus anaerobius.*

Embodiment AB is the culture device of any one of Embodiments U through Y, wherein the nutrient facilitates growth of a *Campylobacter* microorganism.

Embodiment AC is the culture device of Embodiment AA, further comprising an effective amount of a selective agent wherein, when the growth region is reconstituted with a predefined volume of aqueous liquid, the effective amount of selective agent substantially permits germination and growth of the *Campylobacter* microorganism in the culture device and the effective amount of selective agent substantially inhibits growth of a non- *Campylobacter* microorganism.

Embodiment AD is the culture device of any one of Embodiments U through Y, wherein the nutrient facilitates growth of a lactic acid-producing microorganism.

Embodiment AE is the culture device of any one of Embodiments U through AD, further comprising an indicator reagent.

Embodiment AF is the culture device of any one of Embodiments U through AE, further comprising an effective amount of a reducing agent.

Embodiment AG is the culture device of any one of Embodiments U through AF, wherein the enzyme component comprises ascorbic acid oxidase.

Embodiment AH is the culture device of Embodiment AGwherein the enzyme substrate component comprises L-ascorbic acid or a salt thereof.

Embodiment AI is the culture device of Embodiment AH, wherein the second effective amount of L-ascorbic acid or salt thereof disposed in the first coating in the growth region is about 1.5 micromoles/10 cm² to about 15 micromoles/10 cm².

Embodiment AJ is a method of making a culture device for culturing anaerobic microorganisms, the method comprising:
depositing a first coating onto a portion of a first substrate, the first coating being formed using liquid mixture comprising an enzyme component of an enzyme-mediated oxygen depletion system;
drying the first coating;
depositing a second coating onto a second substrate, the second coating comprising an enzyme substrate component of the enzyme-mediated oxygen depletion system; and
positioning the first substrate adjacent the second substrate such that the first coating faces the second coating and such that a growth region disposed between the first substrate and the second substrate overlaps a portion of the first coating and a portion of the second coating.

Embodiment AK is the method of Embodiment AJ, wherein the liquid comprises water.

Embodiment AL is the method of Embodiment AJ, wherein the liquid comprises an organic liquid.

Embodiment AM is the method of any one of Embodiments AJ through AL, wherein the liquid mixture further comprises a cold-water-soluble gelling agent.

Embodiment AN is the method of Embodiment AM as dependent on Embodiment AK, wherein depositing a first coating onto a portion of a first substrate comprises:
mixing the enzyme component and the gelling agent in a predetermined volume of the liquid to form a coating mixture; and
coating the coating mixture onto the first substrate.

Objects and advantages of this invention are further illustrated by the following examples, but the particular materials and amounts thereof recited in these examples, as well as other conditions and details, should not be construed to unduly limit this invention.

### EXAMPLES

**Table 1. List of Materials**

| **Component/Description** | **Source** |
|---|---|
| ALDOL 515 acetate | Biosynth International, Inc.; Itasca, IL |
| Ascorbate oxidase | Calzyme Laboratories, Inc.; San Luis Obispo, CA |
| Brain Heart Infusion | Becton Dickinson; Franklin Lakes, NJ |
| Cefoxitin (C4786) | Sigma-Aldrich Corp.; St. Louis, MO |
| Cycloserine (C6880) | Sigma-Aldrich Corp.; St. Louis, MO |
| L-cysteine | Sigma-Aldrich Corp.; St. Louis, MO |
| FeSO₄ • 7H₂O | Sigma-Aldrich Corp.; St. Louis, MO |
| Guar gum | Rhodia; Bristol, PA |
| Lactobacillus MRS Nutrients (powder); Part number L12-104 | Alpha Biosciences; Baltimore, MD |
| Sodium ascorbate | Sigma-Aldrich Corp.; St. Louis, MO |
| Na₂SO₃ | Sigma-Aldrich Corp.; St. Louis, MO |
| Sodium taurocholate | Alfa Aesar; Ward Hill, MA |
| Sodium thioglycollate | Sigma-Aldrich Corp.; St. Louis, MO |
| MOWIOL PVA-47 (polyvinyl alcohol grade 47-88) | Kuraray America, Inc.; Houston, TX |
| MOWIOL PVA-56 (polyvinyl alcohol grade 56-88) | Kuraray America, Inc.; Houston, TX |
| POVAL PVA-236 (polyvinyl alcohol grade 236) | Kuraray America, Inc.; Houston, TX |
| 2,3,5-Triphenyltetrazolium chloride (TTC) | Biosynth International, Inc.; Itasca, IL |
| Yeast Extract | Becton Dickinson; Franklin Lakes, NJ |

### Example 1. Preparation and use of a self-contained anaerobic environment-generating culture device.

A self-contained anaerobic environment-generating culture device similar to the culture device 10' shown in FIGS. 2 and 3 was constructed. The first substrate consisted of 5 mil (0.127 mm) thickness polyester film (MELINEX Grade 377 biaxially-oriented polyester (PET) film , obtained from DuPont Teijin; Chester, VA). The second substrate consisted of clear polyester (PET) film (0.073 mm thick). Powders comprising nutrients (listed in Table 2), polyvinyl alcohol (PVA), and guar gum were stirred in deionized water to achieve a substantially uniform mixture comprising 3% (w/w) nutrients, 10% (w/v) PVA, and 0.3% (w/v) guar gum, respectively. The mixture was knife-coated onto the first substrate as described in U.S. Patent No. 4,565,783, and dried for 7-8 minutes at 210°F (98.9°C) in a convection oven.. The nutrient layer is knife coated to a thickness that resulted in a target coating weight (after drying) of 0.150 g/24 in² (1.0 mg/cm²). After drying, a polystyrene foam spacer (approximately 0.038 cm thick having a density of approximately 19.3 kg/m³) was adhered to the dried coating on the first substrate via a thin layer of a pressure-sensitive adhesive (98 wt% isooctylacrylate copolymerized with 2 wt% acrylic acid). The spacer comprises 2-inch (5.1-cm) diameter circular openings that defined the hole in the spacer illustrated in FIG. 2. The second substrate was attached to the first substrate along one edge using a double-sided adhesive tape and the devices were cut into approximately 3" (7.6 cm) by 4" (10.1 cm) rectangles similar to those shown in FIG. 2.

**Table 2. Nutrient powder composition used to make coating mixture.**

| **Component** | **Mass Ratio** (%) |
|---|---|
| Sodium Pyruvate | 45 |
| Pancreatic digest of casein | 22.5 |
| Yeast Extract | 15.8 |
| K₂PO₄ • 2H₂O | 9 |
| KH₂PO₄ • 2H₂O | 3.2 |
| Glucose | 4.5 |

A tetrazolium salt (2, 3, 5-tripheyltetrazolium chloride (TTC); final concentration = 20 µg/mL), L-ascorbic acid (final concentration = 1 mg/mL), and ascorbic acid oxidase (final concentration = 4 Units/mL) were added to an aqueous diluent (Butterfield's buffer). The resulting solution was filter-sterilized. A spore stock of *Clostridium sporogenes* ATCC#3584 in water was serially diluted in the aqueous diluent to achieve final suspensions of about 10, 100, and 1000 bacteria per milliliter. One milliliter portions of each suspension were used to inoculate 3M PETRIFILM Aerobic Count (PAC) Plates (3M Company, St. Paul, MN) and the culture devices prepared according to this Example. The PAC plates were incubated in an anaerobic chamber in which a gas sachet (GASPAK EZ Anaerobe Container System Sachets, obtained from Becton, Dickinson and Company, Franklin Lakes, NJ) was used to generate an anaerobic atmosphere. The culture devices of Example 1 were incubated in an incubator having an aerobic (ambient) atmosphere.

The PAC plates and culture devices of Example 1 were incubated at 37 degrees C for 24 hours and were observed for indications of bacterial colonies. Red-colored colonies were observed in all of the PAC plates and all of the culture devices prepared according to Example 1. The number of colonies observed in each plate and culture device was generally consistent with the approximate number of bacteria in the corresponding suspension inoculated into each plate.

### Examples 2-3. Preparation and use of a self-contained anaerobic environment-generating culture device comprising an oxygen indicator.

3M PETRIFILM Aerobic Count (PAC) Plates were obtained from 3M Company; St. Paul, MN. The plates were opened and one milliliter solution of sterile Butterfield's buffer containing the components listed in Table 3 was deposited into the growth area of each of the plates according to the manufacturer's instructions for inoculating the plates. Prior to closing the plates, at least one oxygen sensor (Planar Oxygen Sensor, 5 mm diameter, Part Number 200000023; obtained from Presens Precision Sensing GmnH; Regensburg, DE) was placed onto the hydrated growth area, the top film (i.e., second substrate) was lowered onto the bottom film (i.e., first substrate), and the solution was spread over a circular area in the device using concave side of the spreader provided by the manufacturer of the PAC plates.

**Table 3. Components of the liquid used to hydrate the culture devices.**

| **Culture Device** | **Components of Hydrating Liquid** |
|---|---|
| Example 2 | Sodium ascorbate (10% w/v) |
| | Ascorbic acid oxidase (4 U/mL) |
| Example 3 | Sodium ascorbate (10% w/v) |
| | Ascorbic acid oxidase (4 U/mL) |
| | Polyvinyl alcohol (10% w/v) |
| Control A | Polyvinyl alcohol (10% w/v) |

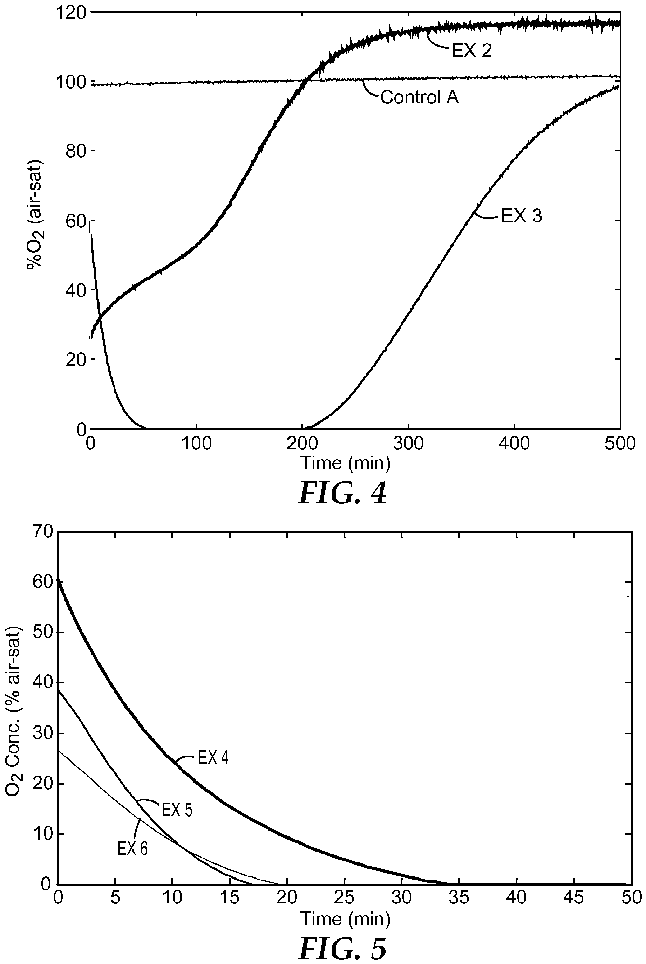

After closing the culture devices, the fluorescence from the sensors was monitored using an OXY-MINI fiberoptic microsensor (Presens Precision Sensing) equipped with vendor-supplied software for the measurement of oxygen. The culture devices were incubated at room temperature (ca. 22 degrees C). The data were collected at 30-second intervals after the culture devices were closed. The results are shown in FIG. 4.

FIG. 4 shows that the plates receiving the enzyme-mediated oxygen depletion system (i.e., sodium ascorbate and ascorbic acid oxidase) reduced the concentration of oxygen in the growth area of the device to at least about 25% or less of air saturated control within 50 minutes after closing the device. Without being bound by theory, it is believed the enzyme-mediated oxygen depletion system initially removed oxygen from the liquid used to inoculate the PETRIFILM plates at a rate that was faster than the re-entry of oxygen into the culture devices of Examples 2 and 3. However, over time, the re-entry of oxygen (e.g., via diffusion through the somewhat oxygen-permeable cover sheet of the PETRIFILM plates) eventually exceeded the rate of consumption by the enzyme reaction, resulting in the return of relatively high concentrations of oxygen into the devices. Without being bound by theory, it appears the PVA slowed the re-entry of oxygen into the devices of Example 3. Nevertheless, these Examples show that oxygen can be removed from the culture devices for a period of time that may permit detectable growth of anaerobic and/or microaerophilic microorganisms.

### Examples 4-6. Preparation and use of a self-contained anaerobic environment-generating culture device comprising a PET second substrate and a substantially dry enzyme.

Self-contained anaerobic environment-generating culture devices were constructed as described in Example 1 with the following exceptions: 1) The inner surface of the second substrate was first coated with a layer of adhesive essentially as described in Example 1 of U.S, Patent No. 4,565,783 and then the adhesive layer was powder coated with guar gum; and 2) The liquid-coating mixture consisted of the nutrients described in Example 1, (30 g/L), powdered guar gum (12 g/L), and 4000 U/L ascorbic acid oxidase.

The resulting culture devices were hydrated with one milliliter of Butterfield's buffer containing sodium ascorbate at the respective concentration shown in Table 4. After hydrating the culture devices, an oxygen sensor was placed into the culture devices, the devices were closed and the liquid was spread over the circular growth region defined by the spacer using the flat side of the spreader provided by the manufacturer of the PAC plates. An oxygen sensor was placed into the growth area of each culture device as described in Examples 2-3. The oxygen saturation of the growth area of the culture devices was measured and recorded as described in Examples 2-3.

**Table 4. Concentration of sodium ascorbate in the aqueous liquid used to prepare the culture devices of Examples 4-6.**

| **Culture Device** | **Hydrating Liquid Composition** |
|---|---|
| Example 4 | Sodium ascorbate (1 mg/mL) |
| Example 5 | Sodium ascorbate (10 mg/mL) |
| Example 6 | Sodium ascorbate (100 mg/mL) |

FIG. 5 shows the oxygen concentrations recorded in Examples 4-6 during the initial 50 minutes following the hydration of the culture devices. Although not shown in FIG. 5, the data were collected for approximately eight days. Representative culture devices from each of Examples 4-6 remained anaerobic (i.e., had no detectable oxygen in the growth region) until at least about 8 days after hydration.

### Examples 7-12. Preparation and use of a self-contained anaerobic environment-generating culture device comprising a substantially dry enzyme and various amounts of substantially dry enzyme substrate.

Culture devices were prepared as described for Examples 4-6 with the exception that the powder used to coat the adhesive layer on the second substrate contained various quantities of sodium ascorbate powder. The concentration of sodium ascorbate in each powder mixture is shown in Table 5.

**Table 5. Concentration of sodium ascorbate in the powder mixture used to coat the second substrate of the culture devices of Examples 7-12.**

| **Culture Device** | **Powder Coating Composition** |
|---|---|
| Example 7 | Guar gum (99% w/w), Sodium ascorbate (1% w/w) |
| Example 8 | Guar gum (98% w/w), Sodium ascorbate (2% w/w) |
| Example 9 | Guar gum (96% w/w), Sodium ascorbate (4% w/w) |
| Example 10 | Guar gum (94% w/w), Sodium ascorbate (6% w/w) |
| Example 11 | Guar gum (92% w/w), Sodium ascorbate (8% w/w) |
| Example 12 | Guar gum (90% w/w), Sodium ascorbate (10% w/w) |

The culture devices were opened and one milliliter solution of sterile Butterfield's buffer was deposited into the growth region of each device as described for examples 4-6. In these examples, the aqueous liquid did not contain sodium ascorbate or ascorbic acid oxidase. After the liquid was deposited into the growth region of the devices, an oxygen sensor was placed into the growth area of each culture device as described in Examples 2-3. The oxygen saturation of the growth area of the culture devices was measured and recorded as described in Examples 2-3. The initial rate of oxygen consumption (i.e., during the first 1-15 minutes after hydrating the culture devices) in the growth region of the hydrated culture devices was calculated and the data are shown in Table 6.

**Table 6. Oxygen consumption rates after hydrating self-contained anaerobic environment-generating culture devices. The data reported are the average and standard deviation of five replicate trials for each culture device formulation. The oxygen consumption rate data are reported as the percentage of oxygen removed per minute from the growth area of the culture device.**

| **Culture Device** | **Oxygen Consumption Rate (Mean ± Std. Deviation)** | **Elapsed Time to Reach 0% O₂ (Mean ± Std. Deviation)** |
|---|---|---|
| Example 7 | 0.68 ± 0.09 | Not attained within 1440 minutes¹ |
| Example 8 | 0.97 ± 0.06 | Not attained within 1440 minutes¹ |
| Example 9 | 2.13 ± 0.20 | 117.2 ± 53.3 minutes |
| Example 10 | 2.92 ± 0.45 | 102.2 ± 25.5 minutes |
| Example 11 | 3.01 ± 0/18 | 55.8 ± 1.4 minutes |
| Example 12 | 3.45 ± 0.28 | 52.6 ± 7.0 minutes |

| | | |
|---|---|---|
| ¹- 0% O₂ was not attained in these culture devices within 24 hours after they were hydrated. Although complete removal of oxygen was not achieved within 24 hours, these devices reached a stable, low concentration of oxygen that could support the growth of microaerophilic bacteria or fungi in the growth region of the devices. | | |

The data in Table 6 show that, in culture devices comprising powder-coated second substrates having up to 6% (w/w) sodium ascorbate, there is an approximate linear relationship between the amount of enzyme substrate (sodium ascorbate) and the oxygen consumption rate in the hydrated culture devices. The data further indicated the O₂ concentrations in Example 7 and Example 8 appeared to plateau at about 50% and 75% of the initial concentrations, respectively.

### Example 13. Effect of gelling agent on the observed oxygen consumption rate in self-contained anaerobic environment-generating culture devices.

Culture devices were prepared according to Example 11 (above) with the exception that, as shown in Table 7, the gelling agent used to prepare the liquid coating mixture (applied to the first substrate) was varied as shown in Table 7. In addition, control culture devices were made with 12 g/L powdered guar gum but did not contain any ascorbic acid oxidase (enzyme) in the liquid coating mixture.

**Table 7. Gel composition for culture devices.**

| **Culture Device** | **Gelling agent(s)** |
|---|---|
| A | Guar gum (12 g/L) |
| B | Guar gum (10.8 g/L) + PVA-56 (1.2 g/L) |
| C | Guar gum (10.8 g/L) + PVA-47 (1.2 g/L) |
| D | Guar gum (10.8 g/L) + PVA-236 (1.2 g/L) |
| Control | Guar gum (12 g/L); no ascorbic acid oxidase |

The culture devices were opened and one milliliter solution of sterile Butterfield's buffer was deposited into the growth region of each device as described for examples 4-6. In this example, the aqueous liquid did not contain sodium ascorbate or ascorbic acid oxidase. After the liquid was deposited into the growth region of the devices, an oxygen sensor was placed into the growth area of each culture device as described in Examples 2-3. The oxygen saturation of the growth area of the culture devices was measured and recorded as described in Examples 2-3 and the initial rate of oxygen consumption in the growth region of the hydrated culture devices was calculated as described in Examples 7-12. The calculated oxygen consumption rates are shown in Table 8.

**Table 8. Oxygen consumption rates after hydrating self-contained anaerobic environment-generating culture devices. The data reported are the average and standard deviation (when applicable) for each culture device formulation.**

| **Culture Device** | **Oxygen Consumption Rate** | **Number of Replicates** |
|---|---|---|
| A | 2.28 ± 0.24 | 3 |
| B | 0.56 ± 0.06 | 3 |
| C | 0.56 | 1 |
| D | 0.75 | 1 |
| Control | 0.49 ± 0.05 | 3 |

The data in Table 8 show that, although there appeared to be a relatively low rate of oxygen consumption in the culture devices containing no ascorbic acid oxidase, the oxygen consumption rate was greater than 4-fold higher in the culture devices in which guar was the only cold-water-soluble gelling agent. Culture devices containing PVA-56 and PVA-47 had slightly higher oxygen consumption rates than the control device that lacked the ascorbic acid oxidase enzyme.

### Example 14. Comparison of oxygen consuming rates in culture devices in which the enzyme was coated and dried to culture devices to which the enzyme was added as a component of the hydrating medium.

One set ("Set E") of culture devices were prepared as described above in Example 11. Another set ("Set F") were prepared similarly, except that the liquid-coating mixture (applied to coat the first substrate) did not contain ascorbic acid oxidase.

The culture devices were opened and one milliliter solution of sterile Butterfield's buffer was deposited into the growth region of each device as described for examples 4-6. In this example, the aqueous liquid used to inoculate the "Set E" culture devices did not contain sodium ascorbate or ascorbic acid oxidase. However, the aqueous liquid used to inoculate the "Set F" culture devices contained ascorbic acid oxidase (at a concentration of 4 Units/mL). After the liquid was deposited into the growth region of the devices, an oxygen sensor was placed into the growth area of each culture device as described in Examples 2-3. The oxygen saturation of the growth area of the culture devices was measured and recorded as described in Examples 2-3 and the initial rate of oxygen consumption in the growth region of the hydrated culture devices was calculated as described in Examples 7-12. The calculated oxygen consumption rates are shown in Table 9.

**Table 9. Oxygen consumption rates after hydrating self-contained anaerobic environment-generating culture devices. The data reported are the average and standard deviation of three replicate trials for each culture device formulation.**

| **Culture Device** | **Oxygen Consumption Rate** |
|---|---|
| E | 3.0 ± 0.23 |
| F | 2.28 ± 0.24 |

The data in Table 9 show that the enzyme (ascorbic acid oxidase) and enzyme substrate (sodium ascorbate) can be coated in the devices in a substantially dry form and, when contacted with a suitable liquid, are capable of rehydrating and participating in an oxygen-consuming reaction.

### Examples 15-16. Self-contained anaerobic environment-generating culture devices and method of use.

Self-contained anaerobic culture devices were prepared according to Example 12 above with the following exceptions: 1) For both Examples 15 and 16, the liquid-coating mixture contained the ingredients shown in Table 10). 2) For Example 16, the adhesive used to coat the second substrate (to a dried coating weight of 0.230 g/24 in² (1.49 mg/cm²)) contained 0.04% (w/w) ALDOL 515 acetate; and 3) For both examples 15 and 16, the coating weight of the liquid-coated mixture applied to the first substrate (after drying) was about 0.257 g/24 in² (1.66 mg/cm²).

A spore stock of *Clostridium sporogenes* ATCC#3584 in water was serially diluted in Butterfield's buffer to achieve final suspensions of about 10 and about 100 bacteria per milliliter. One milliliter aliquots of each suspension was placed into the growth region of individual culture devices and the suspensions were spread across the growth region using the flat side of the spreader provided by the manufacturer of the PAC plates. As a growth control, one milliliter aliquots of each suspension were also used to inoculate individual PAC plates. The suspensions were spread into approximately-5 cm circles in the growth region using the concave side of the spreader provided by the manufacturer of the PAC plates. The culture devices of Examples 15-16 were incubated at 37 degrees C in ambient air for about 24 hours. The inoculated PAC plates were incubated at 37 degrees C for about 24 hours in an anaerobe jar.

**Table 10.**

| Component | Concentration |
|---|---|
| Brain Heart Infusion | 74 g/L |
| Yeast Extract | 10 g/L |
| L-cysteine | 1 g/L |
| FeSO₄ • 7H₂O | 1 g/L |
| Sodium thioglycollate | 0.2 g/L |
| Sodium taurocholate | 1 g/L |
| Na₂SO₃ | 0.5 g/L and 0.25 g/L, respectively |
| Ascorbate oxidase | 4000 Units/L |
| Guar gum | 13 g/L |

After incubation, the culture devices containing the ALDOL 515 acetate (Example 16) were viewed under an ultraviolet light. Brightly-fluorescent *C. sporogenes* colonies were observed in the growth region of the devices.

After incubation, the growth regions of all of the inoculated culture devices containing Na₂SO₃ (Example 16) had characteristic gray-black colonies with gray precipitates surrounding the colonies, indicating growth and production of hydrogen sulfide by the C. *sporogenes.* In addition, gas bubbles were observed adjacent the gray black colonies, further indicating the production of gaseous hydrogen sulfide.

After incubation, the inoculated growth controls all had characteristic red colonies of C. sporogenes growing in the growth region of the PAC plates. The numbers of colonies observed in Example 15 and Example 16 culture devices were within about 0.5 log CFU of the number of colonies observed in the anaerobically-incubated PAC plates.

### Example 17. Regeneration of an anaerobic environment after opening a self-contained anaerobic environment-generating culture device.

Self-contained anaerobic environment-generating culture devices were made according to Example 12 with the following exceptions: 1) Instead of using the powder nutrient mixture (described in Example 1) to make the liquid-coating mixture, dehydrated Lactobacillus MRS nutrients (110 g/L) were used; 2) The adhesive used to coat the second substrate (to a dried coating weight of 0.210 g/24 in² (1.36 mg/cm²)) contained 0.1375% (w/w) 2,3,5-triphenyltetrazolium chloride; and 3) The concentration of guar gum in the liquid-coating mixture was 14 g/L instead of 12 g/L.

Two milliliters of Butterfield's buffer was placed into the growth region of two culture devices. Four oxygen sensors were placed into the growth region of a first culture device ("G"), both culture devices were closed, and the liquid was spread throughout the growth region of both culture devices as described in Example 2. Both culture devices were placed in an environment with ambient levels of oxygen at room temperature (ca. 22 degrees C) and the concentration of oxygen in the growth area of culture device G was measured and recorded as described in Example 2. After a period of 45 minutes, the second culture device ("H") was opened for less than one minute while four oxygen sensors were placed into the growth area. Culture device H was re-closed (i.e., the first and second substrates were brought back into contact with each other with the hydrated growth region sandwiched between them) and the concentration of oxygen in the growth area was measured and recorded as described in Example 2. Care was taken to avoid trapping air bubbles in culture device H when it was re-closed after placing the oxygen sensors therein. A representative portion of the oxygen concentration data for each of the sensors in both of the culture devices is shown in Table 11.

**Table 11. Removal of oxygen from culture devices after activating the enzyme-mediated oxygen depletion system. The data presented here are average values of the four oxygen sensors deposited into the growth region of individual culture devices.**

| **% Oxygen Remaining in Growth Region** | | |
|---|---|---|
| **Time** | **Culture Device G** | **Culture Device H¹** |
| 0 | 89 | 70 |
| 10 | 74 | 59 |
| 20 | 63 | 47 |
| 30 | 52 | 35 |
| 40 | 39 | 22 |
| 50 | 29 | 12 |
| 60 | 20 | 8 |
| 70 | 11 | 3 |
| 80 | 5 | 0 |
| 90 | 2 | 0 |
| 100 | 0 | 0 |

| | | |
|---|---|---|
| ¹ - Time "0" for Culture Device H occurred immediately after the device was reclosed with the oxygen sensors disposed therein. Without being bound by theory, it would be expected that Culture Device H contained less that 40% oxygen when the plates were opened (reference 40-minute time point for Culture Device G), it is likely the oxygen concentration briefly increased before the culture device s were re-closed. | | |

The data show that over half of the oxygen was removed from the culture devices within 40 minutes after the devices were hydrated with the buffer and that all of the oxygen was removed within about 100 minutes (see data from the sensors in device G). The data further show that, upon opening the culture device 45 minutes after hydration, the oxygen concentration in the growth area increases back to about 75% of the initial amount of oxygen in the devices. However, the data further show that, after re-closing the opened culture devices, the enzyme-mediated oxygen depletion system is able to continue removing the oxygen from the growth region and re-establish an anaerobic environment in the culture device. Thus, after activating the oxygen depletion system, the culture devices can be opened (e.g., to streak an inoculum onto the culture medium or place a membrane filter comprising a sample material onto the culture medium) in the presence of oxygen and can re-establish an anaerobic environment.

### Example 18. Use of a self-contained anaerobic environment-generating culture device in a streak-plating culturing technique.

Self-contained anaerobic environment-generating culture devices were prepared according to Example 17. Control devices, having the same nutrient medium composition but lacking the components of the oxygen depletion system were also prepared. Cultures of anaerobic microorganisms were prepared by separately growing *Lactobacillus brevis, L. plantarum, L. paracasei, Pediococcus acidilactici, P. damnosus,* and *P. dextrinicus* for 24-48 hours in Lactobacillus MRS broth in an anaerobic environment. The culture devices were hydrated with two milliliters of Butterfield's buffer as described in Example 17. Individual hydrated culture devices were opened about 30 minutes after they were hydrated and the culture medium in the growth region was streaked with a loopful of one of the cultures. The culture devices were re-closed, taking care not to introduce air bubbles, and were incubated aerobically for up to 7 days at 37 degrees C. The control devices were similarly hydrated and streak-inoculated with a loopful of the cultures. The control devices were incubated in an anaerobic chamber at 37 degrees C. Colonies were typically observed in the culture devices after 24-48 hours of incubation.

After incubation, colonies of each microorganism were observed in typical streak-plate growth patterns in the growth regions of all of the culture devices and all of the control devices.

### Example 19. Use of a self-contained anaerobic environment-generating culture device in a membrane filter culturing technique.

Self-contained anaerobic environment-generating culture devices were prepared according to Example 17. Control devices, having the same nutrient medium composition but lacking the components of the oxygen depletion system were also prepared. The microbial cultures of anaerobic microorganisms prepared for Example 18 were serially diluted in sterile buffer to about 10 culture-forming units (CFU) per milliliter The culture devices were hydrated with two milliliters of Butterfield's buffer as described in Example 17.

Approximately 10 mL of the diluted cultures were filtered through individual sterile membrane filters (0.2 µm SUPORE membrane filters obtained from Pall Corporation; Port Washington, NY). Individual hydrated culture devices were opened about 30 minutes after they were hydrated and one of the filters through which a diluted culture was passed was placed onto the culture medium in the growth region. Care was taken not to trap an air bubble between the membrane filter and the culture medium in the culture device. The culture devices were re-closed, taking care not to introduce air bubbles, and were incubated aerobically for up to 7 days at 37 degrees C. The control devices were similarly hydrated and inoculated with one of the filter membranes through which a diluted culture had been passed. The control devices were incubated in an anaerobic chamber at 37 degrees C. Colonies were typically observed in the culture devices after 24-48 hours of incubation.

After incubation, colonies of each microorganism were observed on the membrane filter that was deposited into the growth region of all of the culture devices and all of the control devices. The numbers of colonies of each organism on the filters were similar in the self-contained anaerobic environment-generating culture devices when compared to the control devices.

### Example 20. Preparation and use of a self-contained anaerobic environment-generating culture device comprising a selective medium for culturing Clostridium difficile.

Self-contained anaerobic environment-generating culture devices were constructed as described in Example 1 with the following exceptions: 1) The inner surface of the second substrate was first coated with a layer of adhesive essentially as described in Example 1 of U.S. Patent No. 4,565,783 and then the adhesive layer was powder coated with a mixture comprising 90% (w/w) guar gum and 10% (w/w) sodium ascorbate; and 2) The adhesive used to coat the second substrate contained 0.4 mg ALDOL 515 acetate per gram of adhesive and was coated onto the second substrate at a coating weight of 1.84 mg/cm² (The coating weight was determined after drying the coating for 5 minutes at 115° F (46° C).); 3) The liquid-coating mixture consisted of the components listed in Table 12; and 4) The liquid-coating mixture was knife-coated onto the first substrate at a coating weight of 2.00 mg/cm² (The coating weight was determined after drying the coating for 8 minutes at 210° F (98.9° C).).

"Control" devices were made similarly except that the liquid-coating mixture did not include the Cefoxitin or the Cycloserine. All of the devices were assembled with foam spacers as described in Example 1.

**Table 12. Composition of selective medium for culturing C. difficile. All components are mixed in deionized water at the listed concentrations.**

| Component | Concentration |
|---|---|
| Brain Heart Infusion | 74 g/L |
| Yeast Extract | 10 g/L |
| L-cysteine | 1 g/L |
| Sodium thioglycollate | 0.2 g/L |
| Sodium taurocholate | 1 g/L |
| Guar gum | 16 g/L |
| Ascorbate oxidase | 4000 Units/L |
| Cefoxitin | 16 mg/L |
| Cycloserine | 500 mg/L |

Individual suspensions of *Clostridium difficile* ATCC 43598, *Escherichia coli* ATCC 25922, *Staphylococcus aureus* ATCC 43598, and *Clostridium sporogenes* were prepared in Butterfield's buffer. The suspensions were diluted in Butterfield's buffer to obtain final suspensions (of the individual organisms) containing approximately 10 colony-forming units (CFU) per milliliter and 100 CFU per milliliter. One milliliter of each suspension was used to inoculate individual culture devices made according to this Example. The culture devices were inoculated by lifting the second substrate to expose the growth area, pipetting the one-milliliter suspension onto the growth area of the first substrate, gently lowering the second substrate until it contacted the suspension and the foam spacer, and gently pressing a flat plastic spreader onto the closed device to spread the liquid suspension throughout the opening of the foam spacer.

All culture devices were placed into an incubator at 37° C and were allowed to incubate in an aerobic atmosphere for about 48 hours. The devices were removed from the incubator and were examined while illuminated with green light (i.e., from LEDs having a peak emission wavelength of about 530 nm) to determine whether microbial colonies were present (as indicated by a fluorescent halo surrounding the colony - indicating the hydrolysis of ALDOL 515 acetate by microorganisms in the colony). The fluorescent colonies were observed and/or photographed using a high-pass red filter with a cut-off of ≥ 590 nm. The qualitative results are shown in Table 13. The results indicate that all of the microorganisms tested were able to grow in the Control devices, indicating the devices supported the growth of facultatively anaerobic microorganisms (e.g., *E. coli* and *S. aureus*) and the growth of obligately-anaerobic microorganisms (e.g., *C. sporogenes* and *C. difficile*). However, only the *C. difficile* microorganisms were able to grow in the devices that contained Cefoxitin and Cycloserine, indicating those devices were selective for *C. difficile* microorganisms.

**Table 13. Growth of various microorganisms in self-contained anaerobic environment-generating culture devices. A "+" indicates that colonies were detected after 48 hours of incubation. A "-" indicates that colonies were not detected after 48 hours of incubation. The number of C. difficile colonies observed in the culture devices with the antibiotics was similar to the number of C. difficile colonies observed in the control plates.**

| Organism | Control Devices (no Cefoxitin or Cycloserine) | Devices with Cefoxitin and Cycloserine |
|---|---|---|
| *C. difficile* | + | + |
| *E. coli* | + | - |
| *S. aureus* | + | - |
| *C. sporogenes* | + | - |

### Example 21. Quantitative comparison of the growth of Clostridium difficile in three different culture environments.

Self-contained anaerobic environment-generating culture devices containing Cefoxitin and Cycloserine were constructed as described in Example 20.

Two types of agar media (Anaerobic blood agar plates, catalog number R01040; and Cycloserine Cefoxitin Fructose agar plates with horse blood, catalog number R01266;) were obtained from Remel Microbiology Products (Lenexa, KS).

A suspension of spores (*Clostridium difficile* ATCC 43598) was made in Butterfield's buffer. A first portion (i.e., the heat-treated (HT) portion) of the suspension was held at 80° C for 20 minutes and a second portion (i.e., the non-treated (NT) portion) was held at ambient temperature.

The HT and NT suspensions were diluted in Butterfield's buffer and 100 microliter aliquots of the diluted suspensions were used to inoculate the surface of each type of the agar plates using sterile spreaders. After inoculation, the agar plates were placed into an anaerobe jar containing a sachet that was activated to generate an anaerobic environment. The anaerobe jar was sealed and placed into an incubator having an aerobic atmosphere at 37° C.

A diluting micropipettor was used to dilute 100 microliter aliquots of the diluted suspensions into one-milliliter volumes, which were used to inoculate the self-contained anaerobic environment-generating culture devices containing Cefoxitin and Cycloserine. After inoculation, the culture devices were placed into the aerobic atmosphere of the incubator at 37° C.

All inoculated agar Petri plates and all inoculated self-contained anaerobic environment-generating culture devices were incubated for 48 hours. After incubation, the bacterial colonies in each plate or device were counted. The number of colonies was multiplied by the dilution factor to obtain a calculation of the number of spores in the original suspensions. The calculated data are shown in Table 14.

**Table 14. Calculated CFU per milliliter of spore suspension based on the number of colonies observed in the agar plates and self-contained anaerobic environment-generating culture devices.**

| Culture Device | Heat-treated Spores | Non-treated Spores |
|---|---|---|
| Anaerobic blood agar | 1.46 × 10⁵ CFU/mL | 1.06 × 10⁶ CFU/mL |
| Cycloserine Cefoxitin Fructose agar | 1.18 × 10⁷ CFU/mL | 3.5 × 10⁸ CFU/mL |
| Self-contained anaerobic environment-generating culture devices¹ | 7.7 × 10⁶ CFU/mL | 3.42 × 10⁸ CFU/mL |

| | | |
|---|---|---|
| ¹ - Results reported for the self-contained anaerobic environment-generating culture devices are the average of the colony counts obtained from two identical culture devices. | | |

The results indicate that the recovery and growth of *C*. *difficile* colonies in the self-contained anaerobic environment-generating culture devices was similar to the recovery and growth of *C. difficile* colonies in the Cycloserine Cefoxitin Fructose agar plates that were incubated in an anaerobic atmosphere. In addition, the results indicate that the recovery and growth of *C. difficile* colonies in the self-contained anaerobic environment-generating culture devices was better than the recovery and growth of *C. difficile* colonies in the Anaerobic blood agar plates that were incubated in an anaerobic atmosphere.

### Example 22. Preparation and use of a self-contained anaerobic environment-generating culture device for culturing Lactic acid Producing Bacteria.

A self-contained anaerobic environment-generating culture device similar to the culture device 10' shown in FIGS. 2 and 3 was constructed. The first substrate consisted of 5 mil (0.127 mm) thickness polyester film (MELINEX Grade 377 biaxially-oriented polyester (PET) film, obtained from DuPont Teijin; Chester, VA). The components listed in Table 15 were added to deionized water (1L) and the mixture was stirred. Sodium hydroxide (1N) was then added to the stirred mixture to adjust the pH to about 6.5. Guar gum (14 g) was then added and stirring was continued to achieve a substantially uniform mixture. The mixture was knife-coated onto the first substrate as described in U.S. Patent No. 4,565,783, and dried for 7-8 minutes at 210° F (98.9° C) in a convection oven. The coating weight (after drying) was 0.4 g/24 in² (2.6 mg/cm²). After drying, a polystyrene foam spacer (approximately 0.038 cm thick having a density of approximately 19.3 kg/m³) was adhered to the dried coating on the first substrate via a thin layer of a pressure-sensitive adhesive (98 wt% isooctylacrylate copolymerized with 2 wt% acrylic acid). The spacer comprised a 2-inch (5.1-cm) diameter circular opening illustrated in FIG. 2. The circular opening defined the growth region of the device.

The second substrate consisted of clear polyester (PET) film (0.073 mm thick). The inner surface of the second substrate was coated with a first coating formulation containing TTC in the same second substrate adhesive described in Examples 4-6. The dried coating weight in this construction was 0.2 g/24 in² (1.3 mg/cm²) and the TTC concentration in the dried coating was about 0.02 mg/ in² (0.003 mg/cm²). The adhesive layer of the second substrate was then powder coated with a homogeneous mixture of guar (previously combined with 0.01% CAB-O-SIL fused silica) (90% w/w) and sodium ascorbate (10% w/w).

The second substrate was attached to the first substrate along one edge using a double-sided adhesive tape and the assembled devices were cut into approximately 3" (7.6 cm) by 4" (10.1 cm) rectangles so that the circular opening was positioned in approximately the center of the device similar to those shown in FIG. 2. The second substrate served as the cover sheet for the self-contained anaerobic environment-generating culture device.

The self-contained anaerobic environment-generating culture devices were inoculated with a single microbial sample selected from Bacterial Strain Set A (Table 16). Suspensions of each sample were diluted in Butterfield's Buffer water to yield final suspensions (of the individual organisms) containing about 100 CFU per mL (dilution A), 1000 CFU per mL (dilution B), and 10000 CFU per mL (dilution C). One milliliter of each suspension was used to inoculate individual culture devices made according to this Example. The culture devices were inoculated by lifting the second substrate to expose the growth area, pipetting the one-milliliter suspension onto the growth area of the first substrate, gently lowering the second substrate until it contacted the suspension and the foam spacer, and gently pressing a flat plastic spreader onto the closed device to spread the liquid suspension throughout the opening of the foam spacer.

After inoculation, the self-contained anaerobic environment-generating culture devices were incubated at 32° C for 60 hours in an aerobic incubator. The red-colored bacterial colonies in each culture device were counted by visual examination at 24 hours and at 60 hours. The results are presented in Tables 17 ad 18.

**Table 15. Composition used to make coating mixture for first substrate of Example 22.**

| **Component** | **Amount** | **Source** |
|---|---|---|
| Dextrose | 40 g | Becton Dickinson; Franklin Lakes, NJ |
| Pancreatic digest of gelatin | 20 g | Alpha Biosciences; Baltimore, MD |
| Beef Extract | 16 g | Alpha Biosciences; Baltimore, MD |
| Sodium acetate | 10 g | J.T. Baker; Center Valley, PA |
| Yeast extract | 8 g | Alpha Biosciences; Baltimore, MD |
| Ferric ammonium citrate | 4 g | Spectrum Chemical; New Brunswick, NJ |
| Potassium phosphate dibasic | 4 g | J.T. Baker; Center Valley, PA |
| Polysorbate 80 | 2 g | Amresco; Solon, OH |
| Magnesium sulfate | 0.4 g | J.T. Baker; Center Valley, PA |
| Manganese sulfate | 0.1 g | J.T. Baker; Center Valley, PA |
| Succinic acid, disodium salt | 6.5 g | Alfa Aesar; Ward Hill, MA |
| Monosodium succinate | 8.4 g | Alfa Aesar; Ward Hill, MA |
| Bromocresol green | 2 g | Sigma-Aldrich Corp; St. Louis, MO |
| Ascorbate oxidase | 4000 Units | Calzyme Laboratories; San Louis Obispo, CA |

**Table 16. Bacterial Strain Set A (used in Example 22)**

| **Component** |
|---|
| *Leuconostoc mesenteroides* ssp *dextranicum* |
| *Streptococcus salivarius* |
| *Leuconostoc mesenteroides* ssp *mesenteroides* |
| *Streptococcus alactolyticus* |
| *Lactobacillus brevis* |
| *Staphylococcus epidermis* |
| *Lactobacillus alimentarius* |
| *Pediococcus damnosus* |
| *Steptococcus equinus* |

**Table 17. Example 22 (24 hour incubation)**

| | **Colony Count in CFU (24 hr Incubation)** | | |
|---|---|---|---|
| **Component** | **Dilution A** | **Dilution B** | **Dilution C** |
| *Leuconostoc mesenteroides* ssp *dextranicum* | 150 | TNTC | TNTC |
| *Streptococcus salivarius* | 5 | 5 | 5 |
| *Leuconostoc mesenteroides* ssp *mesenteroides* | 100 | TNTC | TNTC |
| *Streptococcus alactolyticus* | 50 | 200 | TNTC |
| *Lactobacillus brevis* | 250 | TNTC | TNTC |
| *Staphylococcus epidermis* | 0 | 0 | 0 |
| *Lactobacillus alimentarius* | 0 | 0 | 0 |
| *Pediococcus damnosus* | 0 | 0 | 10 |
| *Steptococcus equinus* | 250 | TNTC | TNTC |

| | | | |
|---|---|---|---|
| *TNTC = 'too numerous to count' | | | |

**Table 18. Example 22 (60 hour incubation)**

| | **Colony Count in CFU (60 hr Incubation)** | | |
|---|---|---|---|
| **Component** | **Dilution A** | **Dilution B** | **Dilution C** |
| *Leuconostoc mesenteroides* ssp *dextranicum* | 200 | TNTC | TNTC |
| *Streptococcus salivarius* | 10 | 1 | 10 |
| *Leuconostoc mesenteroides* ssp *mesenteriodes* | 200 | TNTC | TNTC |
| *Streptococcus alactolyticus* | 200 | TNTC | TNTC |
| *Lactobacillus brevis* | 250 | TNTC | TNTC |
| *Staphylococcus epidermis* | 70 | TNTC | TNTC |
| *Lactobacillus alimentarius* | 150 | TNTC | TNTC |
| *Pediococcus damnosus* | 10 | 0 | 10 |
| *Steptococcus equinus* | TNTC | TNTC | TNTC |

| | | | |
|---|---|---|---|
| *TNTC = 'too numerous to count' | | | |

### Example 23. Preparation and use of a self-contained anaerobic environment-generating culture device for culturing Lactic acid Producing Bacteria.

The same self-contained anaerobic environment-generating culture device as described in Example 22 was prepared with the one exception that after the components listed in Table 15 were added to deionized water (1L) and the mixture was stirred, sodium hydroxide (1N) was then added to the stirred mixture to adjust the pH to about 5.4 (instead of pH 6.5 as in Example 22).

The self-contained anaerobic environment-generating culture devices were inoculated with a single microbial sample selected from Bacterial Strain Set A (Table 16). Suspensions of each sample were diluted in Butterfield's Buffer water to yield final suspensions (of the individual organisms) containing about 100 CFU per mL (dilution A), 10 CFU per mL (dilution D), and 1 CFU per mL (dilution E). One milliliter of each suspension was used to inoculate individual culture devices made according to this Example. The culture devices were inoculated by lifting the second substrate to expose the growth area, pipetting the one-milliliter suspension onto the growth area of the first substrate, gently lowering the second substrate until it contacted the suspension and the foam spacer, and gently pressing a flat plastic spreader onto the closed device to spread the liquid suspension throughout the opening of the foam spacer.

After inoculation, the self-contained anaerobic environment-generating culture devices were incubated at 32° C for 72 hours in an aerobic incubator. The culture devices were evaluated at 72 hours for the presence of red-colored bacteria colonies (Positive) or absence of red-colored bacteria colonies (Negative). Associated colony gas bubbles were also observed for the devices inoculated with *Leuconostoc mesenteriodes* ssp *dextranicum* and *Leuconostoc mesenteroides* ssp *mesenteroides.* T he results are presented in Table 19.

**Table 19. Detection of colonies using the self-contained anaerobic environment-generating culture devices of Example 23**

| | **Colonies Detected (72 hr Incubation)** | | |
|---|---|---|---|
| | **Dilution A** | **Dilution D** | **Dilution E** |
| *Leuconostoc mesenteroi4des* ssp *dextranicum* | Positive | Positive | Positive |
| *Streptococcus salivarius* | Positive | Positive | Positive |
| *Leuconostoc mesenteroides* ssp *mesenteroides* | Positive | Positive | Positive |
| *Streptococcus alactolyticus* | Positive | Positive | Positive |
| *Lactobacillus brevis* | Positive | Positive | Positive |
| *Staphylococcus epidermis* | Positive | Positive | Positive |
| *Lactobacillus alimentarius* | Positive | Positive | Positive |
| *Pediococcus damnosus* | Positive | Positive | Positive |
| *Steptococcus equinus* | Positive | Positive | Positive |

### Example 24. Preparation and use of a self-contained anaerobic environment-generating culture device for culturing Lactic acid Producing Bacteria.

The same self-contained anaerobic environment-generating culture devices as described in Example 22 were prepared with the one exception that the bromocresol green pH indicator listed in Table 15 was replaced with chlorophenol red (0.5 g, Sigma-Aldrich). The self-contained anaerobic environment-generating culture devices were inoculated with a single microbial sample of either *Leuconostoc mesenteroides* spp *dextranicum* or *Leuconostoc mesenteroides* ssp *mesenteroides.* Suspensions of each sample were diluted in Butterfield's Buffer water to yield final suspensions (of the individual organisms) containing about 100 CFU per mL. The inoculations were conducted as described in Example 22 and the devices were incubated at 32° C for 48 hours in an aerobic incubator. Following incubation, the culture devices were visually evaluated for bacterial colonies. Colonies (red-colored) with associated gas bubbles were observed in all of the culture devices.

### Example 25. Preparation and use of a self-contained anaerobic environment-generating culture device for culturing Lactic acid Producing Bacteria.

The same self-contained anaerobic environment-generating culture devices as described in Example 24 were prepared with the one exception that the bromocresol green pH indicator listed in Table 15 was replaced with chlorophenol red (0.25 g). The self-contained anaerobic environment-generating culture devices were inoculated with a single microbial sample of either *Leuconostoc mesenteroides* spp *dextranicum* or *Leuconostoc mesenteroides* ssp *mesenteroides.* Suspensions of each sample were diluted in Butterfield's Buffer water to yield final suspensions (of the individual organisms) containing about 100 CFU per mL. The inoculations were conducted as described in Example 22 and the devices were incubated at 32° C for 48 hours in an aerobic incubator. Following incubation, the culture devices were visually evaluated for bacterial colonies. Colonies (red-colored) with associated gas bubbles were observed in all of the culture devices.

### Example 26. Preparation and use of a self-contained anaerobic environment-generating culture device for culturing Lactic acid Producing Bacteria.

The same self-contained anaerobic environment-generating culture devices as described in Example 22 were prepared with two exceptions. First, the bromocresol green pH indicator listed in Table 15 was replaced with chlorophenol red (0.5 g). Second, after the components listed in Table 15 were added to deionized water (1L) and the mixture was stirred, sodium hydroxide (IN) was then added to the stirred mixture to adjust the pH to about 5.4 (instead of pH 6.5 as in Example 22). The self-contained anaerobic environment-generating culture devices were inoculated with a single microbial sample of either *Leuconostoc mesenteroides* spp *dextranicum* or *Leuconostoc mesenteroides* ssp *mesenteroides.* Suspensions of each sample were diluted in Butterfield's Buffer water to yield final suspensions (of the individual organisms) containing about 100 CFU per mL. The inoculations were conducted as described in Example 22 and the devices were then incubated at 32° C for 48 hours in an aerobic incubator. Following incubation, the culture devices were visually evaluated for bacterial colonies. Colonies (red-colored) with associated gas bubbles were observed in all of the culture devices.

### Example 27. Preparation and use of a self-contained anaerobic environment-generating culture device for culturing Lactic acid Producing Bacteria.

The same self-contained anaerobic environment-generating culture devices as described in Example 22 were prepared with two exceptions. First, the bromocresol green pH indicator listed in Table 15 was replaced with chlorophenol red (0.25 g). Second, after the components listed in Table 15 were added to deionized water (1L) and the mixture was stirred, sodium hydroxide (IN) was then added to the stirred mixture to adjust the pH to about 5.4 (instead of pH 6.5 as in Example 22). S The self-contained anaerobic environment-generating culture devices were inoculated with a single microbial sample of either *Leuconostoc mesenteroides* spp *dextranicum* or *Leuconostoc mesenteroides* ssp *mesenteroides.* Suspensions of each sample were diluted in Butterfield's Buffer water to yield final suspensions (of the individual organisms) containing about 100 CFU per mL. The inoculations were conducted as described in Example 22 and the devices were incubated at 32° C for 48 hours in an aerobic incubator. Following incubation, the culture devices were visually evaluated for bacterial colonies. Colonies (red-colored) with associated gas bubbles were observed in all of the culture devices.

### Example 28. Preparation and use of a self-contained microaerophilic environment-generating culture device for culturing Campylobacter Bacteria.

A self-contained microaerophilic environment-generating culture device similar to the culture device 10' shown in FIGS. 2 and 3 was constructed. The first substrate consisted of 5 mil (0.127 mm) thickness polyester film (MELINEX Grade 377 biaxially-oriented polyester (PET) film, obtained from DuPont Teijin; Chester, VA). The components listed in Table 20 were added to deionized water (1L) and the mixture was stirred. Guar gum (14 g) was then added and stirring was continued to achieve a substantially uniform mixture. The mixture was knife-coated onto the first substrate as described in U.S. Patent No. 4,565,783, and dried for 7-8 minutes at 210 °F (98.9 °C) in a convection oven. The coating weight (after drying) was 0.25 g/24 in² (1.6 mg/cm²). After drying, a polystyrene foam spacer (approximately 0.038 cm thick having a density of approximately 19.3 kg/m³) was adhered to the dried coating on the first substrate via a thin layer of a pressure-sensitive adhesive (98 wt% isooctylacrylate copolymerized with 2 wt% acrylic acid). The spacer comprised a 2-inch (5.1-cm) diameter circular opening illustrated in FIG. 2. The circular opening defined the growth region of the device.

The second substrate consisted of clear polyester (PET) film (0.073 mm thick). The inner surface of the second substrate was coated with a first coating formulation containing TTC in the same second substrate adhesive described in Examples 4-6. The dried coating weight in this construction was 0.2 g/24 in² (1.3 mg/cm²) and the TTC concentration in the dried coating was about 0.02 mg/ in² (0.003 mg/cm²). The adhesive layer of the second substrate was then powder coated with a homogeneous mixture of guar (previously combined with 0.01% CAB-O-SIL fused silica) (97.5% w/w) and sodium ascorbate (2.5% w/w).

The second substrate was attached to the first substrate along one edge using a double-sided adhesive tape and the assembled devices were cut into approximately 3" (7.6 cm) by 4" (10.1 cm) rectangles so that the circular opening was positioned in approximately the center of the device similar to those shown in FIG. 2. The second substrate served as the cover sheet for the self-contained microaerophilic environment-generating culture device.

Individual suspensions of *Campylobacter jejuni* ATCC 29428 and *Campylobacter jejuni* ATCC 33291 were prepared in Butterfield's buffer. The suspensions were serially diluted in Butterfield's buffer to obtain final suspensions (of the individual organisms) having concentrations that provided CFU counts of about 10-100. One milliliter of each suspension was used to inoculate individual culture devices made according to this Example. The culture devices were inoculated by lifting the second substrate to expose the growth area, pipetting the one-milliliter suspension onto the growth area of the first substrate, gently lowering the second substrate until it contacted the suspension and the foam spacer, and gently pressing a flat plastic spreader onto the closed device to spread the liquid suspension throughout the opening of the foam spacer.

After inoculation, the self-contained anaerobic environment-generating culture devices were incubated at 41.5° C for 48 hours in an aerobic incubator. Following incubation, the red-colored bacterial colonies in each culture device were counted by visual examination. The number of counted colonies (mean value from two replicates) was multiplied by the dilution factor to obtain a calculation of the number of cells in the original suspensions (CFU/mL). The calculated data is presented in Table 21.

As a comparative reference, agar plates were also inoculated with individual *Campylobacter jejuni* ATCC 29428 and *Campylobacter jejuni* ATCC 33291 suspensions. The agar plates were prepared using Campy Blood-Free Selective Medium (CBFSM) (catalog #C03-102), Alpha Biosciences, Baltimore, MD). The CBFSM (44.5 g) was sequentially suspended in 1L of purified water; boiled for one minute to dissolve the medium; autoclaved at 121° C for 15 minutes; and cooled to 45-50° C. The cooled medium (15-20 mL) was then poured into a sterile Petri dish. A 100 microliter aliquot of the serially diluted sample was spread plated onto the agar plate. The inoculated plate was then placed in an anaerobic chamber together with a gas-generating sachet (GASPAK EZ Campy Container System Sachets, obtained from Becton, Dickinson and Company, Franklin Lakes, NJ) that provided a microaerophilic atmosphere during the incubation period. The agar plate was incubated under these conditions at 41.5° C degrees for 48 hours. The number of counted colonies (mean value from two replicates) on each agar plate was multiplied by the dilution factor to obtain a calculation of the number of cells in the original suspensions (CFU/mL). The calculated data is presented in Table 21.

**Table 20. Composition used to make coating mixture for first substrate of Example 28.**

| **Component** | **Amount** | **Source** |
|---|---|---|
| Heart Infusion | 19 g | Hardy Diagnostics; Santa Maria, CA |
| Lactalbumin hydrolysate | 10.3 g | Alfa Aesar; Ward Hill, MA |
| Casamino acids | 8.4 g | Amresco; Solon, OH |
| Hemin | 0.02 g | Sigma-Aldrich Corp; St. Louis, MO |
| alpha-Ketoglutaric acid | 2 g | Spectrum Chemical; New Brunswick, NJ |
| Bile salts #3 | 0.7 g | Becton Dickinson; Franklin Lakes, NJ |
| Sodium carbonate | 1.2 g | Becton Dickinson; Franklin Lakes, NJ |
| Sodium pyruvate | 2 g | Sigma-Aldrich Corp; St. Louis, MO |
| Tryptone | 5 g | Alpha Biosciences; Baltimore, MD |
| Ascorbate oxidase | 4000 Units | Calzyme Laboratories; San Louis Obispo, CA |

**Table 21. Calculated CFU per milliliter of cells in the original suspensions based on the number of colonies observed in the agar plates and the self-contained microaerophilic environment-generating culture devices of Example 28.**

| | self-contained microaerophilic environment-generating culture device of Example 28 | CBFSM Petri Plate |
|---|---|---|
| *Campylobacter jejuni ATCC 29428* | 2.6 X 10⁷ CFU/mL | 7.5 X 10⁶ CFU/mL |
| *Campylobacter jejuni ATCC 33291* | 1.0 X 10⁷ CFU/mL | 8.4 X 10⁶ CFU/mL |

### Example 29. Preparation and use of a self-contained microaerophilic environment-generating culture device for culturing Campylobacter Bacteria.

The same self-contained microaerophilic environment-generating culture device as described in Example 28 was prepared with the one exception that the components listed in Table 22 (instead of the components in Table 20) were used to prepare the coating on the first substrate and the dried coating weight was 0.26 g/24 in² (1.7 mg/cm2).

Individual suspensions of *Campylobacter jejuni* ATCC 29428, *Campylobacter jejuni* ATCC 33291, *Campylobacter coli* ATCC 33559, and *Campylobacter coli* ATCC 43476 were prepared in Butterfield's buffer. The suspensions were serially diluted in Butterfield's buffer to obtain final suspensions (of the individual organisms) having concentrations that provided CFU counts of about 10-100. One milliliter of each suspension was used to inoculate individual culture devices prepared according to Example 28. The culture devices were inoculated by lifting the second substrate to expose the growth area, pipetting the one-milliliter suspension onto the growth area of the first substrate, gently lowering the second substrate until it contacted the suspension and the foam spacer, and gently pressing a flat plastic spreader onto the closed device to spread the liquid suspension throughout the opening of the foam spacer.

After inoculation, the self-contained microaerophilic environment-generating culture devices were incubated at 41.5° C for 48 hours in an aerobic incubator. Following incubation, the red-colored bacterial colonies in each culture device were counted by visual examination. The number of counted colonies (mean value from two replicates) was multiplied by the dilution factor to obtain a calculation of the number of cells in the original suspensions (CFU/mL). The calculated data is presented in Table 23.

As a comparative reference, CBFSM agar plates (described above) were inoculated with suspensions (one organism per plate) of *Campylobacter jejuni* ATCC 29428, *Campylobacter jejuni* ATCC 33291, *Campylobacter coli* ATCC 33559, and *Campylobacter coli* ATCC 43476 and processed according to the method described in Example 28. The number of counted colonies (mean value from two replicates) on each agar plate was multiplied by the dilution factor to obtain a calculation of the number of cells in the original suspensions (CFU/mL). The calculated data is presented in Table 23.

**Table 22. Composition used to make coating mixture for first substrate of Example 29.**

| **Component** | **Amount** |
|---|---|
| Peptone from Meat | 20 g |
| Lactalbumin hydrolysate | 10 g |
| Yeast extract | 10 g |
| Sodium chloride | 10 g |
| alpha-Ketoglutaric acid | 2 g |
| Sodium pyruvate | 1 g |
| Sodium carbonate | 1.2 g |
| Hemin | 0.02 g |
| Ferrous sulfate (heptahydrate) | 0.3 g |
| Ascorbate oxidase | 4000 Units |

**Table 23. Calculated CFU per milliliter of cells in the original suspensions based on the number of colonies observed in the agar plates and the self-contained microaerophilic environment-generating culture devices of Example 29.**

| | self-contained microaerophilic environment-generating culture device of Example 29 | CBFSM Petri Plate |
|---|---|---|
| *Campylobacter jejuni ATCC 9428* | 2. 1 X 10⁵ CFU/mL | 6.0 X 10⁶ CFU/mL |
| *Campylobacter jejuni ATCC 33291* | 1.2 X 10⁷ CFU/mL | 5.9 X 10⁷ CFU/mL |
| *Campylobacter coli ATCC 33559* | 3.2 X 10⁶ CFU/mL | 1.0 X 10⁷ CFU/mL |
| *Campylobacter coli ATCC 43476* | 4.4 X 10⁷ CFU/mL | 2.6 X 10⁷ CFU/mL |

The foregoing detailed description and examples have been given for clarity of understanding only. No unnecessary limitations are to be understood therefrom. The invention is not limited to the exact details shown and described, for variations obvious to one skilled in the art will be included within the invention defined by the claims.

All headings are for the convenience of the reader and should not be used to limit the meaning of the text that follows the heading, unless so specified.

Various modifications may be made without departing from the scope of the invention. These and other embodiments are within the scope of the following claims.

## Claims

1. A method of detecting a microorganism in a sample, the method comprising:
contacting a growth region of a self-contained anaerobic environment-generating culture device with a predefined volume of aqueous liquid wherein, prior to the step of contacting the growth region with the predefined volume, the growth region comprises a dry, cold-water-soluble gelling agent and a fermentable carbohydrate;
wherein the growth region is defined as a volume disposed between the inner surfaces of a first substrate and a second substrate;
wherein the dry, cold water-soluble gelling agent is disposed as a coating on an inner surface of the first substrate;
depositing an effective amount of an enzyme component of an enzyme-mediated oxygen depletion system into the growth region;
contacting the growth region with a sample;
incubating the culture device for a period of time sufficient to permit formation of a microbial colony; and
detecting the presence or absence of a microbial colony, wherein detecting the absence of a microbial colony comprises the absence of gas bubbles associated with the metabolism of the fermentable carbohydrate in the growth medium;
further comprising depositing an effective amount of an enzyme substrate component of the enzyme-mediated oxygen depletion system into the growth region.

2. The method of claim 1, wherein contacting the growth region with the aqueous liquid comprises contacting the growth region with the sample.

3. The method of claim 1, wherein the step of contacting the growth region with the sample is not simultaneous with the step of contacting the growth region with the predefined volume of aqueous liquid.

4. The method of claim 3, wherein the step of contacting the growth region with the sample occurs after the step of contacting the growth region with the predefined volume of aqueous liquid.

5. The method of claim 3, wherein the step of contacting the growth region with the sample occurs before the step of contacting the growth region with the predefined volume of aqueous liquid.

6. The method of any one of the preceding claims, wherein incubating the culture device for a period of time sufficient to permit formation of a microbial colony comprises incubating the culture device for the period of time in an aerobic gaseous environment.

7. The method of any one of the preceding claims,
wherein a first dry composition is coated on a first substrate of the culture device and a second dry composition is coated onto a second substrate of the culture device;
wherein, when the first composition, second composition, and the aqueous liquid are placed in fluidic communication in the growth region of the culture device, they form an aqueous mixture that comprises a first concentration of dissolved oxygen;
wherein, while the device is held in an aerobic environment for less than or equal to about 120 minutes after the liquid mixture is formed, the first concentration of dissolved oxygen is reduced by the enzyme-mediated oxygen depletion system to a second concentration of dissolved oxygen that does not substantially inhibit growth of a microaerotolerant microorganism.

8. The method of any one of claims 1 through 6:
wherein a first dry composition is coated on a first substrate of the culture device and a second dry composition is coated onto a second substrate of the culture device;
wherein, when the first composition, second composition, and the aqueous liquid are placed in fluidic communication in the growth region of the culture device, they form an aqueous mixture that comprises a first concentration of dissolved oxygen;
wherein, while the device is held in an aerobic environment for less than or equal to about 120 minutes after the liquid mixture is formed, the first concentration of dissolved oxygen is reduced by the enzyme-mediated oxygen depletion system to a second concentration of dissolved oxygen that does not substantially inhibit growth of a microaerophilic microorganism.

9. The method of any one of claims 1 through 6:
wherein a first dry composition is coated on a first substrate of the culture device and a second dry composition is coated onto a second substrate of the culture device;
wherein, when the first composition, second composition, and the aqueous liquid are placed in fluidic communication in the growth region of the culture device, they form an aqueous mixture that comprises a first concentration of dissolved oxygen;
wherein, while the device is held in an aerobic environment for less than or equal to about 120 minutes after the liquid mixture is formed, the first concentration of dissolved oxygen is reduced by the enzyme-mediated oxygen depletion system to a second concentration of dissolved oxygen that does not substantially inhibit growth of an obligately-anaerobic microorganism.

10. A culture device for enumerating colonies of microorganisms, the device comprising:
a first substrate having opposing inner and outer surfaces;
a second substrate having opposing inner and outer surfaces;
a growth region disposed between the inner surfaces of the first and second substrates;
an effective amount of a substantially dry enzyme component of an enzyme-mediated oxygen depletion system, the first effective amount disposed in a first coating in the growth region;
an effective amount of a substantially dry enzyme substrate component of the enzyme-mediated oxygen depletion system, the second effective amount disposed in a second coating in the growth region; and
a dry, cold-water-soluble gelling agent disposed in the growth region;
wherein the first substrate and second substrate are substantially non-transmissible to gaseous oxygen;
wherein components that are substantially dry have a water content no greater than about the water content of the dehydrated coating once it has been permitted to equilibrate with the ambient environment; and
wherein the first and second coatings are placed in fluid communication when an aqueous liquid is deposited between the first and second substrates.

11. The culture device of claim 10 wherein, when the effective amounts of the enzyme component and the enzyme substrate component are brought into fluid communication in the growth region using a predefined volume of aqueous liquid, the enzyme component and the enzyme substrate component are capable of reacting to reduce a first dissolved-oxygen concentration in the aqueous liquid to a second dissolved-oxygen concentration that is substantially lower than the first dissolved-oxygen concentration.

12. The culture device of claim 10 or claim 11, further comprising a nutrient that facilitates growth of a microorganism.

13. The culture device of any one of claims 10 through 12, further comprising an effective amount of a reducing agent.

14. The culture device of any one of claims 10 through 13, wherein the enzyme component comprises ascorbic acid oxidase.

## Patentansprüche

1. Ein Verfahren zum Nachweisen eines Mikroorganismus in einer Probe, wobei das Verfahren Folgendes umfasst:
Inkontaktbringen eines Wachstumsbereichs einer in sich geschlossenen, eine anaerobe Umgebung erzeugenden Kulturvorrichtung mit einem vorbestimmten Volumen an wässriger Flüssigkeit, wobei der Wachstumsbereich vor dem Schritt des Inkontaktbringens des Wachstumsbereichs mit dem vorbestimmten Volumen ein trockenes, kaltwasserlösliches Geliermittel und ein fermentierbares Kohlenhydrat umfasst;
wobei der Wachstumsbereich definiert ist als ein Volumen, das zwischen den Innenoberflächen eines ersten Substrats und eines zweiten Substrats angeordnet ist;
wobei das trockene, kalt wasserlösliche Geliermittel als Beschichtung auf einer Innenoberfläche des ersten Substrats angeordnet ist;
Abscheiden einer wirksamen Menge eines Enzymbestandteils eines enzymvermittelten Sauerstoffverarmungssystems in den Wachstumsbereich;
Inkontaktbringen des Wachstumsbereichs mit einer Probe;
Inkubieren der Kulturvorrichtung für einen Zeitraum, der ausreichend ist, um die Bildung einer mikrobiellen Kolonie zu ermöglichen; und
Nachweisen des Vorhandenseins oder Fehlens einer mikrobiellen Kolonie, wobei das Nachweisen des Fehlens einer mikrobiellen Kolonie das Fehlen von Gasblasen umfasst, die mit dem Stoffwechsel des fermentierbaren Kohlenhydrats im Wachstumsmedium verbunden sind; ferner
umfassend Abscheiden einer wirksamen Menge eines Enzymsubstratbestandteils des enzymvermittelten Sauerstoffverarmungssystems in den Wachstumsbereich.

2. Das Verfahren nach Anspruch 1, wobei das Inkontaktbringen des Wachstumsbereichs mit der wässrigen Flüssigkeit das Inkontaktbringen des Wachstumsbereichs mit der Probe umfasst.

3. Das Verfahren nach Anspruch 1, wobei der Schritt des Inkontaktbringens des Wachstumsbereichs mit der Probe nicht gleichzeitig mit dem Schritt des Inkontaktbringens des Wachstumsbereichs mit dem vorbestimmten Volumen der wässrigen Flüssigkeit erfolgt.

4. Das Verfahren nach Anspruch 3, wobei der Schritt des Inkontaktbringens des Wachstumsbereichs mit der Probe nach dem Schritt des Inkontaktbringens des Wachstumsbereichs mit dem vorbestimmten Volumen einer wässrigen Flüssigkeit erfolgt.

5. Das Verfahren nach Anspruch 3, wobei der Schritt des Inkontaktbringens des Wachstumsbereichs mit der Probe vor dem Schritt des Inkontaktbringens des Wachstumsbereichs mit dem vorbestimmten Volumen der wässrigen Flüssigkeit erfolgt.

6. Das Verfahren nach einem der vorhergehenden Ansprüche, wobei das Inkubieren der Kulturvorrichtung für einen Zeitraum, der ausreichend ist, um die Bildung einer mikrobiellen Kolonie zu ermöglichen, das Inkubieren der Kulturvorrichtung für den Zeitraum in einer aeroben gasförmigen Atmosphäre umfasst.

7. Das Verfahren nach einem der vorstehenden Ansprüche,
wobei eine erste trockene Zusammensetzung auf ein erstes Substrat der Kulturvorrichtung aufgebracht wird und eine zweite trockene Zusammensetzung auf ein zweites Substrat der Kulturvorrichtung aufgebracht wird;
wobei, wenn die erste Zusammensetzung, die zweite Zusammensetzung und die wässrige Flüssigkeit in fluidischer Verbindung im Wachstumsbereich der Kulturvorrichtung angeordnet sind, sie ein wässriges Gemisch bilden, das eine erste Konzentration von gelöstem Sauerstoff umfasst;
wobei, während die Vorrichtung für weniger als oder gleich etwa 120 Minuten nach Bildung des flüssigen Gemisches in einer aeroben Umgebung gehalten wird, die erste Konzentration an gelöstem Sauerstoff durch das enzymatisch vermittelte Sauerstoffverarmungssystem auf eine zweite Konzentration an gelöstem Sauerstoff reduziert wird, die das Wachstum eines mikroaerotoleranten Mikroorganismus nicht wesentlich hemmt.

8. Das Verfahren nach einem der Ansprüche 1 bis 6:
wobei eine erste trockene Zusammensetzung auf ein erstes Substrat der Kulturvorrichtung aufgebracht wird und eine zweite trockene Zusammensetzung auf ein zweites Substrat der Kulturvorrichtung aufgebracht wird;
wobei, wenn die erste Zusammensetzung, die zweite Zusammensetzung und die wässrige Flüssigkeit in fluidischer Verbindung im Wachstumsbereich der Kulturvorrichtung angeordnet sind, sie ein wässriges Gemisch bilden, das eine erste Konzentration von gelöstem Sauerstoff umfasst;
wobei, während die Vorrichtung für weniger als oder gleich etwa 120 Minuten nach Bildung des flüssigen Gemisches in einer aeroben Umgebung gehalten wird, die erste Konzentration an gelöstem Sauerstoff durch das enzymatisch vermittelte Sauerstoffverarmungssystem auf eine zweite Konzentration an gelöstem Sauerstoff reduziert wird, die das Wachstum eines mikroaerophilen Mikroorganismus nicht wesentlich hemmt.

9. Das Verfahren nach einem der Ansprüche 1 bis 6:
wobei eine erste trockene Zusammensetzung auf ein erstes Substrat der Kulturvorrichtung aufgebracht wird und eine zweite trockene Zusammensetzung auf ein zweites Substrat der Kulturvorrichtung aufgebracht wird;
wobei, wenn die erste Zusammensetzung, die zweite Zusammensetzung und die wässrige Flüssigkeit in fluidischer Verbindung im Wachstumsbereich der Kulturvorrichtung angeordnet sind, sie ein wässriges Gemisch bilden, das eine erste Konzentration von gelöstem Sauerstoff umfasst;
wobei, während die Vorrichtung für weniger als oder gleich etwa 120 Minuten nach Bildung des flüssigen Gemisches in einer aeroben Umgebung gehalten wird, die erste Konzentration an gelöstem Sauerstoff durch das enzymatisch vermittelte Sauerstoffverarmungssystem auf eine zweite Konzentration an gelöstem Sauerstoff reduziert wird, die das Wachstum eines zwingend anaeroben Mikroorganismus nicht wesentlich hemmt.

10. Eine Kulturvorrichtung zum Zählen von Kolonien von Mikroorganismen, wobei die Vorrichtung Folgendes umfasst:
ein erstes Substrat mit gegenüberliegenden Innen- und Außenoberflächen;
ein zweites Substrat mit gegenüberliegenden Innen- und Außenoberflächen;
einen Wachstumsbereich, der zwischen den Innenoberflächen des ersten und zweiten Substrats angeordnet ist;
eine wirksame Menge eines im Wesentlichen trockenen Enzymbestandteils eines enzymvermittelten Sauerstoffverarmungssystems, wobei die erste wirksame Menge in einer ersten Beschichtung im Wachstumsbereich angeordnet ist;
eine wirksame Menge eines im Wesentlichen trockenen Enzymbestandteils eines enzymvermittelten Sauerstoffverarmungssystems, wobei die zweite wirksame Menge in einer zweiten Beschichtung im Wachstumsbereich angeordnet ist; und
ein trockenes, kaltwasserlösliches Geliermittel, das im Wachstumsbereich angeordnet ist;
wobei das erste Substrat und das zweite Substrat im Wesentlichen nicht auf gasförmigen Sauerstoff übertragbar sind;
wobei Bestandteile, die im Wesentlichen trocken sind, einen Wassergehalt aufweisen, der nicht größer ist als der Wassergehalt der dehydrierten Beschichtung, nachdem er mit der Umgebung ins Gleichgewicht kommen konnte; und
wobei die erste und zweite Beschichtung in fluidische Verbindung gebracht werden, wenn eine wässrige Flüssigkeit zwischen dem ersten und zweiten Substrat abgeschieden wird.

11. Die Kulturvorrichtung nach Anspruch 10, wobei, wenn die wirksamen Mengen des Enzymbestandteils und des Enzymsubstratbestandteils im Wachstumsbereich unter Verwendung eines vorbestimmten Volumens an wässriger Flüssigkeit in fluidische Verbindung gebracht werden, der Enzymbestandteil und der Enzymsubstratbestandteil in der Lage sind zu reagieren, um eine erste gelöste Sauerstoffkonzentration in der wässrigen Flüssigkeit auf eine zweite gelöste Sauerstoffkonzentration zu reduzieren, die wesentlich niedriger als die erste gelöste Sauerstoffkonzentration ist.

12. Die Kulturvorrichtung nach Anspruch 10 oder Anspruch 11, ferner umfassend einen Nährstoff, der das Wachstum eines Mikroorganismus erleichtert.

13. Die Kulturvorrichtung nach einem der Ansprüche 10 bis 12, die ferner eine wirksame Menge eines Reduktionsmittels umfasst.

14. Die Kulturvorrichtung nach einem der Ansprüche 10 bis 13, wobei der Enzymbestandteil Ascorbinsäure-Oxydase umfasst.

## Revendications

1. Procédé de détection d'un micro-organisme dans un échantillon, le procédé comprenant :
la mise en contact d'une région de croissance d'un dispositif de culture autonome générant un environnement anaérobie avec un volume prédéfini de liquide aqueux dans lequel, avant l'étape de mise en contact de la région de croissance avec le volume prédéfini, la région de croissance comprend un agent gélifiant sec, soluble dans l'eau froide et un glucide fermentable ;
dans lequel la région de croissance est définie comme un volume disposé entre les surfaces internes d'un premier substrat et d'un deuxième substrat ;
dans lequel l'agent gélifiant sec, soluble dans l'eau froide est disposé en tant que revêtement sur une surface interne du premier substrat ;
le dépôt d'une quantité efficace d'un composant d'enzyme d'un système d'appauvrissement en oxygène médié par enzyme dans la région de croissance ;
la mise en contact de la région de croissance avec un échantillon ;
l'incubation du dispositif de culture pendant un laps de temps suffisant pour permettre la formation d'une colonie microbienne ; et
la détection de la présence ou de l'absence d'une colonie microbienne, dans lequel la détection de l'absence d'une colonie microbienne comprend l'absence de bulles de gaz associées au métabolisme du glucide fermentable dans le milieu de croissance ; comprenant en outre le dépôt d'une quantité efficace d'un composant de substrat d'enzyme du système d'appauvrissement en oxygène médié par enzyme dans la région de croissance.

2. Procédé selon la revendication 1, dans lequel la mise en contact de la région de croissance avec le liquide aqueux comprend la mise en contact de la région de croissance avec l'échantillon.

3. Procédé selon la revendication 1, dans lequel l'étape de mise en contact de la région de croissance avec l'échantillon n'est pas simultanée à l'étape de mise en contact de la région de croissance avec le volume prédéfini de liquide aqueux.

4. Procédé selon la revendication 3, dans lequel l'étape de mise en contact de la région de croissance avec l'échantillon se produit après l'étape de mise en contact de la région de croissance avec le volume prédéfini de liquide aqueux.

5. Procédé selon la revendication 3, dans lequel l'étape de mise en contact de la région de croissance avec l'échantillon se produit avant l'étape de mise en contact de la région de croissance avec le volume prédéfini de liquide aqueux.

6. Procédé selon l'une quelconque des revendications précédentes, dans lequel l'incubation du dispositif de culture pendant un laps de temps suffisant pour permettre la formation d'une colonie microbienne comprend l'incubation du dispositif de culture pendant le laps de temps dans un environnement gazeux aérobie.

7. Procédé selon l'une quelconque des revendications précédentes,
dans lequel une première composition sèche est revêtue sur un premier substrat du dispositif de culture et une deuxième composition sèche est revêtue sur un deuxième substrat du dispositif de culture ;
dans lequel, lorsque la première composition, la deuxième composition, et le liquide aqueux sont placés en communication fluidique dans la région de croissance du dispositif de culture, ils forment un mélange aqueux qui comprend une première concentration d'oxygène dissous ;
dans lequel, alors que le dispositif est maintenu dans un environnement aérobie pendant un temps inférieur ou égal à environ 120 minutes après que le mélange liquide est formé, la première concentration d'oxygène dissous est réduite par le système d'appauvrissement en oxygène médié par enzyme à une deuxième concentration d'oxygène dissous qui n'inhibe essentiellement pas la croissance d'un micro-organisme micro-aérotolérant.

8. Procédé selon l'une quelconque des revendications 1 à 6 :
dans lequel une première composition sèche est revêtue sur un premier substrat du dispositif de culture et une deuxième composition sèche est revêtue sur un deuxième substrat du dispositif de culture ;
dans lequel, lorsque la première composition, la deuxième composition, et le liquide aqueux sont placés en communication fluidique dans la région de croissance du dispositif de culture, ils forment un mélange aqueux qui comprend une première concentration d'oxygène dissous ;
dans lequel, alors que le dispositif est maintenu dans un environnement aérobie pendant un temps inférieur ou égal à environ 120 minutes après que le mélange liquide est formé, la première concentration d'oxygène dissous est réduite par le système d'appauvrissement en oxygène médié par enzyme à une deuxième concentration d'oxygène dissous qui n'inhibe essentiellement pas la croissance d'un micro-organisme micro-aérophile.

9. Procédé selon l'une quelconque des revendications 1 à 6 :
dans lequel une première composition sèche est revêtue sur un premier substrat du dispositif de culture et une deuxième composition sèche est revêtue sur un deuxième substrat du dispositif de culture ;
dans lequel, lorsque la première composition, la deuxième composition, et le liquide aqueux sont placés en communication fluidique dans la région de croissance du dispositif de culture, ils forment un mélange aqueux qui comprend une première concentration d'oxygène dissous ;
dans lequel, alors que le dispositif est maintenu dans un environnement aérobie pendant un temps inférieur ou égal à environ 120 minutes après que le mélange liquide est formé, la première concentration d'oxygène dissous est réduite par le système d'appauvrissement en oxygène médié par enzyme à une deuxième concentration d'oxygène dissous qui n'inhibe essentiellement pas la croissance d'un micro-organisme obligatoirement anaérobie.

10. Dispositif de culture pour le dénombrement de colonies de micro-organismes, le dispositif comprenant :
un premier substrat ayant des surfaces interne et externe opposées ;
un deuxième substrat ayant des surfaces interne et externe opposées ;
une région de croissance disposée entre les surfaces internes des premier et deuxième substrats ;
une quantité efficace d'un composant d'enzyme essentiellement sec d'un système d'appauvrissement en oxygène médié par enzyme, la première quantité efficace disposée dans un premier revêtement dans la région de croissance ;
une quantité efficace d'un composant de substrat d'enzyme essentiellement sec du système d'appauvrissement en oxygène médié par enzyme, la deuxième quantité efficace disposée dans un deuxième revêtement dans la région de croissance ; et
un agent gélifiant sec, soluble dans l'eau froide disposé dans la région de croissance ;
dans lequel le premier substrat et le deuxième substrat sont essentiellement non transmissibles à l'oxygène gazeux ;
dans lequel les composants qui sont essentiellement secs ont une teneur en eau n'excédant pas environ la teneur en eau du revêtement déshydraté une fois qu'on l'a laissé s'équilibrer avec l'environnement ambiant ; et
dans lequel les premier et deuxième revêtements sont placés en communication fluidique lorsqu'un liquide aqueux est déposé entre les premier et deuxième substrats.

11. Dispositif de culture selon la revendication 10 dans lequel, lorsque les quantités efficaces du composant d'enzyme et du composant de substrat d'enzyme sont amenées en communication fluidique dans la région de croissance en utilisant un volume prédéfini de liquide aqueux, le composant d'enzyme et le composant de substrat d'enzyme sont susceptibles de réagir pour réduire une première concentration en oxygène dissous dans le liquide aqueux à une deuxième concentration en oxygène dissous qui est essentiellement inférieure à la première concentration en oxygène dissous.

12. Dispositif de culture selon la revendication 10 ou la revendication 11, comprenant en outre un nutriment qui facilite la croissance d'un micro-organisme.

13. Dispositif de culture selon l'une quelconque des revendications 10 à 12, comprenant en outre une quantité efficace d'un agent réducteur.

14. Dispositif de culture selon l'une quelconque des revendications 10 à 13, dans lequel le composant d'enzyme comprend de l'acide ascorbique-oxydase.
